(19) Europäisches Patentamt
European Patent Office
Office européen des brevets

(11) **EP 4 160 183 B1**

(12) **EUROPEAN PATENT SPECIFICATION**

(45) Date of publication and mention
of the grant of the patent:
**31.12.2025 Bulletin 2026/01**

(21) Application number: **21817325.0**

(22) Date of filing: **28.05.2021**

(51) International Patent Classification (IPC):
**G01N 15/00** *(2024.01)*      **G01N 33/53** *(2006.01)*

(52) Cooperative Patent Classification (CPC):
**G01N 15/1459; G01N 15/1031; G01N 15/1429;**
**G01N 33/5094; G01N 33/56972; G01N 35/00722;**
G01N 2015/016; G01N 2015/1006;
G01N 2015/1486; G01N 2035/0091

(86) International application number:
**PCT/JP2021/020467**

(87) International publication number:
**WO 2021/246334 (09.12.2021 Gazette 2021/49)**

(54) **MULTI-PURPOSE AUTOMATED BLOOD CELL COUNTING DEVICE**

AUTOMATISIERTE MEHRZWECK-BLUTZELLENZÄHLVORRICHTUNG

DISPOSITIF DE NUMÉRATION DE CELLULES SANGUINES AUTOMATISÉ POLYVALENT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **02.06.2020 JP 2020095973**

(43) Date of publication of application:
**05.04.2023 Bulletin 2023/14**

(73) Proprietor: **So, Shimbu**
**Tokyo, 108-0075 (JP)**

(72) Inventor: **So, Shimbu**
**Tokyo, 108-0075 (JP)**

(74) Representative: **Murgitroyd & Company**
**165-169 Scotland Street**
**Glasgow G5 8PL (GB)**

(56) References cited:
**WO-A1-2007/145333      WO-A1-2019/131578**
**JP-A- 2014 521 052      JP-A- 2017 090 158**
**JP-A- 2020 073 924      JP-A- H02 500 297**
**US-A1- 2018 259 504**

• **SO, SHIMBU ET AL.: "An analytical formula of**
**simple lymphocyte to monitor immunokinetics",**
**JAPANESE JOURNAL OF CLINICAL**
**LABORATORY AUTOMATION, vol. 42, no. 4, 1**
**January 2017 (2017-01-01), JP**
**, pages 470, XP009532878, ISSN: 0286-1607**

**Description**

## TECHNICAL FIELD

The present invention relates to a multi-purpose automated blood cell counting device that can perform follow-up examination on changes in immunodynamics.

## BACKGROUND

Currently, in the clinical field, there are many cases in which immunodynamics are examined by examining the interrelationship between lymphocytes. Examining the dynamics of immune subgroups enables predicting autoimmune diseases, allergic diseases, the presence of cancer, the transition of cancer status, and the detailed information of cancer. In addition, examination of immunodynamics is not only beneficial for early detection of diseases, but can also be used as an indicator for evaluating therapeutic effects and side effects in preventing and treating diseases, and has become an essential examination item for early detection and early treatment of diseases in the modern medical field.

As basic indicators in examination of immunodynamics, it is needless to say that helper T cells, which are called the control tower of immunity, are important, but other than this, indicator information such as killer T cells, B cells, NK cells, NKT cells, etc. is also important. For example, based on the above-mentioned basic indicators, it is possible to derive detailed information that are strongly related to immune symptoms with Th1 cells, Th2 cells, Th17 cells, regulatory T cells, etc., and this is beneficial for early detection and early treatment of diseases.

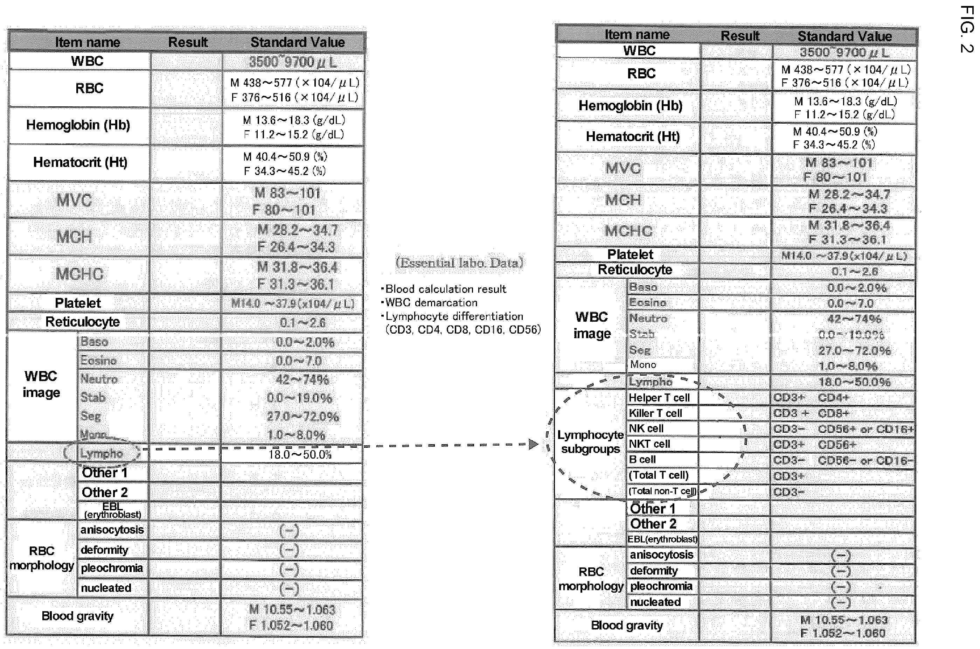

Meanwhile, there are two types of blood sampling tests (for example, in Japan), which are an in-hospital test (general examination item) and an out-of-hospital test performed in examination facilities, etc. (an examination including items described in Patent Document 1). From 40 years ago until today, in a white blood cell clinical examination (in-hospital test), the mainstream examinations have been an overall examination report of four types of fraction of granulocytes (neutrophils, eosinophils, basophils, and mononuclear cells) and lymphocytes. In these clinical examinations, the fractions of the dynamic state of granulocytes are shown, but the fractions of the states of the lymphocyte subgroups are not shown (for example, see the table on the left in FIG. 2). Currently, showing the fractions of the states of the lymphocyte subgroups (for example, see the table on the right in FIG. 2) is very complicated and troublesome, as disclosed in, for example, Patent Document 1, and an examination of individual blood collection tubes must be performed.

Conventionally, for many medical institutions to acquire information related to a more detailed immune status (information of the fractions of the dynamic state of granulocytes and the fractions of the dynamic state of the lymphocyte subgroups), a request needed to be made to specialized institutions such as Tokyo Koshueisei Laboratories, Inc. to perform a customized examination of individual blood collection tubes (e.g., complicated methods such as a PCR method), and this was very expensive. In particular, in order to investigate the dynamic state of the lymphocyte subgroups, a large number of items had to be examined simultaneously, and therefore, in a clinical stage in which individual items are required, an examination using conventional techniques is highly unreasonable. In addition, the complex and expensive examination methods up to the present time can indeed provide information of many types of items, but all of them are merely static examinations for specific parts of immunity. That is, in the clinical field, indicator information of helper T cells, killer T cells, B cells, NKs cells, NKT cells, etc. can not be obtained immediately, and information related to the dynamics of the entire lymphocyte subgroups is difficult to obtain, thereby causing major problems for medical staff and patients.

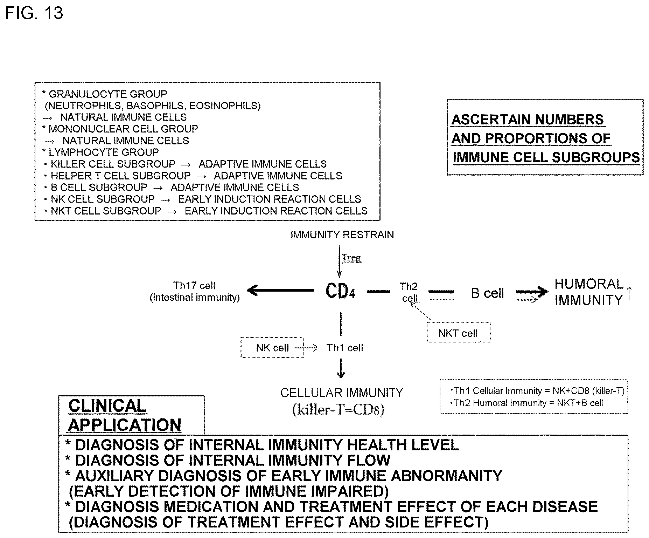

As shown in FIG. 13, indicator information (a portion of the immune cell subgroups) related to helper T cells, killer T cells, B cells, NK cells, NKT cells, etc. is directly related to many pathological conditions and constitutional changes (cellular changes). That is, immediately acquiring indicator information of helper T cells, killer T cells, B cells, NK cells, NKT cells, etc. enables determining and treating many pathological conditions and constitutional changes in an early stage.

Recently, in order to implement a small examination device for Point of Care Testing (POCT) particularly in a blood cell count examination device that counts blood cells in a blood sample and measures hemoglobin concentration, sensors such as a blood cell count sensor, a hemoglobin concentration measurement sensor, and a blood sample volume weighing sensor, which are main parts of the small examination device, are reduced in size and integrated to acquire a blood cell count examination chip having a disposable structure for the main body of the device, and at the same time, a blood cell count examination device that uses this blood cell count examination chip is known (see Patent Document 1). In addition, an immunity evaluation method, an immunity evaluation device, and an immunity evaluation program, which evaluate immunity using immune cell markers corresponding to a plurality of immune cells contained in collected blood, as well as an information recording medium in which the immunity evaluation program is recorded are known (see Patent Document 2). However, a device that can immediately acquire indicator information of helper T cells, killer T cells, B cells, NK cells, NKT cells, etc. is not known yet.

In modern society, in order to prevent the spread of infectious diseases caused by unknown viruses that rapid spread as a result of increased mobility of people and globalization, it is important to immediately determine the immune status of people and find the truth of the matter. In the current clinical field, although mechanical devices such as automatic blood cell measuring devices and flow cytometers are used, complicated methods such as a PCR method needs to be preformed in order to investigate information of Th1 cells, Th2 cells, Th17 cells, regulatory T cells, etc. which are strongly related to immune symptoms. Moreover, information of related cells acquired by complicated methods such as a PCR method indicates a static state, and it is difficult to acquire information related to immunodynamics from this kind of information. Therefore, for staff in the clinical field, there is a need for development of a device that provides information of Th1 cells, Th2 cells, Th17 cells, regulatory T cells, etc. which are strongly related to immune symptoms, to provide information of immunodynamics at an earlier stage, without performing complicated methods such as a PCR method. Further the disclosure of US 2018/259504 A1 may be helpful for understanding the present invention. This document refers to a method capable of testing an immune state in more detail and in a simple and inexpensive manner to identify diseases such as cancer and to identify side-effects of treatment with an anticancer agent and the like, effects of immunotherapy, a state of prognosis, and the like. The method includes: a step of calculating the total number of white blood cells serving as immune cells in a body of a subject and the number of each of immune cell subgroups based on test information on blood counts of blood components and white blood cell images of the subject and analysis information on the CD classification of monoclonal antibodies that bind to surface antigens of white blood cells; and a step of analyzing the immune state of the subject based on a proportion and a change in the numbers of the immune cell subgroups.

## CITATION LIST

### PATENT LITERATURE

Patent Document 1: JP 2008-89382 A

Patent Document 2: WO2007/145333 A1

## SUMMARY OF INVENTION

### TECHNICAL PROBLEM

In view of the circumstances described above, an object of the present disclosure is to enable medical staff to immediately provide immunity information related to the examinees, specifically information of immunodynamics. More specifically, the object of the present disclosure is to provide a multi-parameter automatic blood cell counting device that can derive detailed information related to Th1 cells, Th2 cells, Th17 cells, regulatory T cells, etc., which are strongly related to immune symptoms, and that can create and display an image of information of immunodynamics.

### SOLUTION TO PROBLEM

In order to solve the problem described above, the present invention refers to a multi-parameter automatic blood cell counting device according to claim 1. Advantageous embodiments may include features of depending claims.

### EFFECTS OF INVENTION

According to the present disclosure, it is possible to provide a multi-parameter automatic blood cell counting device that can immediately acquire information related to immunodynamics, accurately comprehend the immune status, and apply it to prevent the decline of immunity and progress of disease. In particular, using the multi-parameter automatic blood cell counting device of the present disclosure enables immediately comprehending information related to immunodynamics, leading to early detection and treatment of infectious diseases, etc., preventing the spread of infection.

## BRIEF DESCRIPTION OF DRAWINGS

FIG. 1 is a schematic view of a multi-parameter automatic blood cell counting device according to a first embodiment.

FIG. 2 is a diagram showing fractions of the statuses of lymphocyte subgroups.

FIG. 3A is a diagram showing an immunodynamic pattern formed by an immunodynamic change analyzer according to the first embodiment.

FIG. 3B is a diagram showing an immunodynamic model formed by the immunodynamic change analyzer according to the first embodiment.

FIG. 4 is a schematic view of the immunodynamic change analyzer according to the first embodiment.

FIG. 5 shows diagrams of proportions of the number of CD4 antigens, the number of CD8 antigens, the number of NK cells, the number of NKT cells, and the number of B cells of infected persons displayed on a display.

FIG. 6A is a diagram showing comparative influenza infection pattern standard data that is displayed on the display by a pattern formation module.

FIG. 6B is a diagram showing immune cell basic data of a subject that is displayed on the display by the pattern formation module.

FIG. 7 shows a table in which components of patients with gastric cancer and healthy persons are compared, and (A) is a pie chart showing the proportions of the components of the white blood cell subgroups of the patients with gastric cancer, (B) is a pie chart showing the proportion of only the lymphocyte subgroups of the patients with gastric cancer, (C) is a pie chart showing the proportions of the components of the white blood cell subgroups of the healthy persons, and (D) is a pie chart showing the proportion of only the lymphocyte subgroups of the healthy persons.

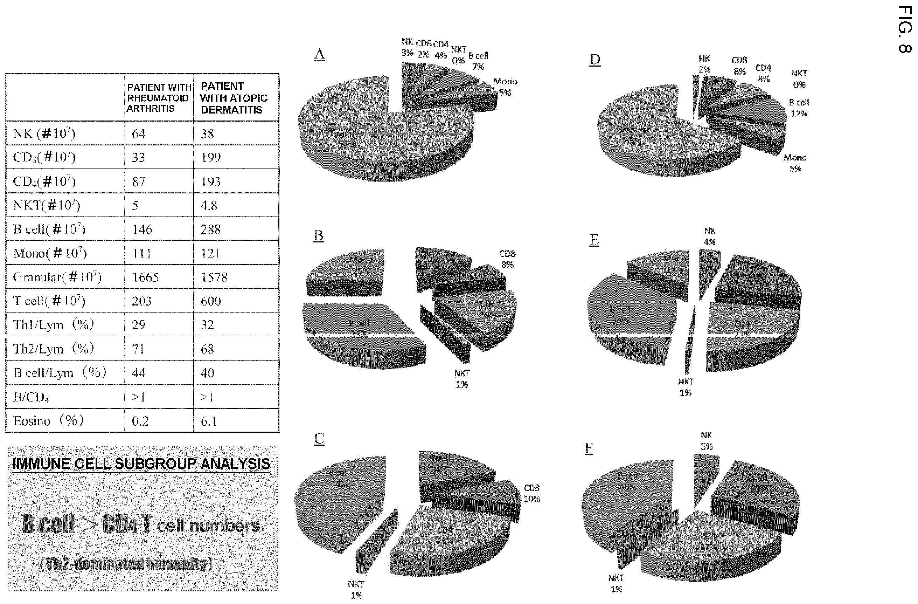

FIG. 8 shows a table in which components of patients with rheumatoid arthritis and patients with atopic dermatitis are compared, and (A) is a pie chart showing the proportions of the components of the white blood cell subgroups of the patients with rheumatoid arthritis, (B) is a pie chart showing the proportions of the lymphocyte subgroups and the single cells of the patients with rheumatoid arthritis, (C) is a pie chart showing the proportion of only the lymphocyte subgroups of the patients with rheumatoid arthritis, (D) is a pie chart showing the proportions of the components of the white blood cell subgroups of the patients with atopic dermatitis, (E) is a pie chart showing the proportions of the lymphocyte subgroups and the single cells of the patients with atopic dermatitis, and (F) is a pie chart showing the proportions of only the lymphocyte subgroups of the patients with atopic dermatitis.

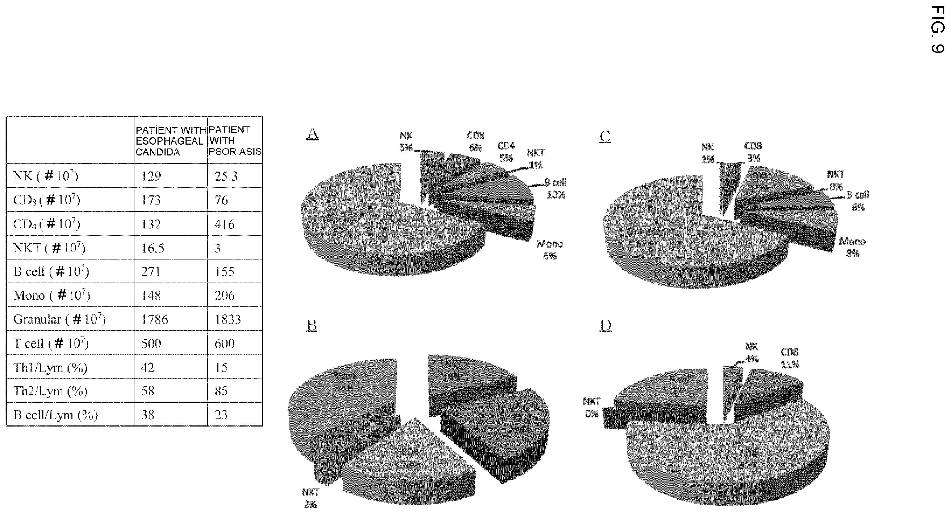

FIG. 9 shows a table in which components of patients with esophageal candida and patients with psoriasis are compared, and (A) is a pie chart showing the proportions of the components of the white blood cell subgroups of the patients with esophageal candida, (B) is the pie chart showing the proportion of only the lymphocyte subgroups of the patients with esophageal candida, (C) is a pie chart showing the proportions of the components of the white blood cell subgroups of the patients with psoriasis, and (D) is a pie chart showing the proportion of only the lymphocyte subgroups of the patients with psoriasis.

FIG. 10 is a diagram showing an immunodynamic model formed by an immunodynamic correlator of the first embodiment.

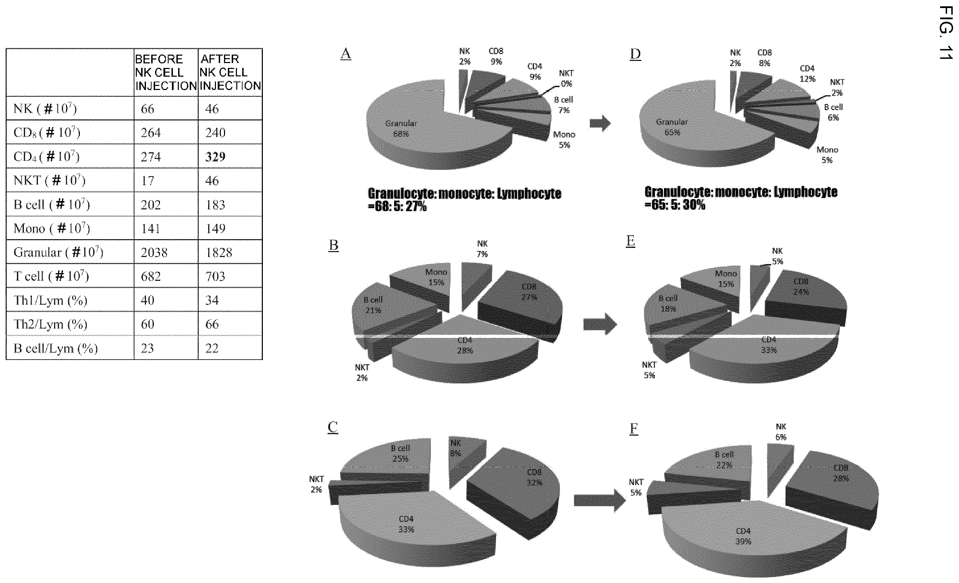

FIG. 11 shows a table in which components in a subject before NK cells are administered and in the subject after NK cells are administered are compared, and (A) is a pie chart showing the proportions of the components of the white blood cell subgroups of the subject before NK cells are administered, (B) is a pie chart showing the proportions of the monocytes and lymphocyte subgroups of the subject before NK cells are administered, (C) is a pie chart showing the proportion of only the lymphocyte subgroups of the subject before NK cells are administered, (D) is a pie chart showing the proportions of the components of the white blood cell subgroups of the subject after NK cells are administered, (E) is a pie chart showing the proportions of the monocytes and lymphocyte subgroups of the subject after NK cells are administered, and (F) is a pie chart showing the proportion of only the lymphocyte subgroups of the subject after NK cells are administered.

FIG. 12 is a diagram showing an immunodynamic model providing an immunity correlation from an influenza infection pattern 1 (during influenza infection) to an influenza recovery pattern 1 (during influenza recovery).

FIG. 13 is a schematic view showing pathological conditions and cell changes which are related with immune cell subgroups.

**DESCRIPTION OF EMBODIMENTS**

Hereinafter, embodiments of a multi-parameter automatic blood cell counting device 1 according to the present disclosure will be described with reference to the drawings.

First Embodiment

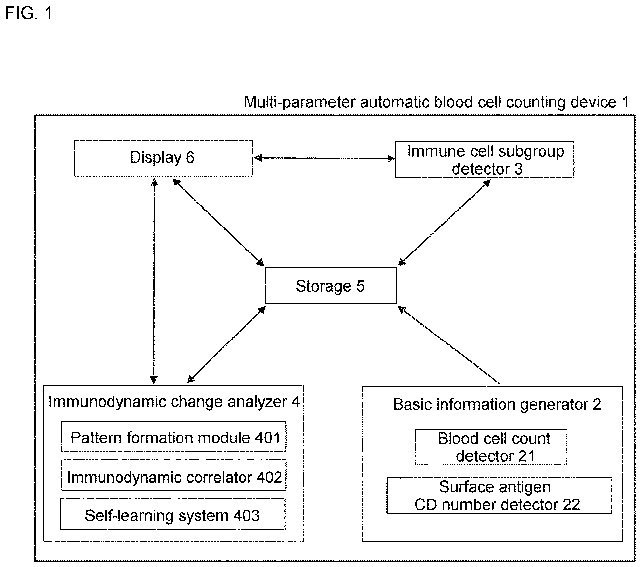

As shown in FIG. 1, a multi-parameter automatic blood cell counting device 1 of a first embodiment includes a basic information generator 2, an immune cell subgroup detector 3, an immunodynamic change analyzer 4, a storage 5, and a display 6.

The basic information generator 2 generates the number of white blood cells, a white blood cell image (white blood cell fraction information), an analysis result of a surface antigen CD number of white blood cells (immune cells), etc.

In the present embodiment, the basic information generator 2 acquires the number of white blood cells, a white blood cell image (white blood cell fraction information), and an analysis result of a surface antigen CD number of white blood cells (immune cells). As shown in FIG. 1, the basic information generator 2 is composed of a blood cell count detector 21 and a surface antigen CD number detector 22.

In the present embodiment, the blood cell count detector 21 is composed of a blood cell counting device that performs treatments such as dilution, hemolysis, and staining of blood and optically or electrically counts blood cells, or a blood cell count receiver that receives blood cell counts from other blood cell counting devices. That is, the blood cell count detector 21 may acquire the number of white blood cells and a white blood cell image (white blood cell fraction information).

When the blood cell count detector 21 is a blood cell counting device, the blood cell count detector 21 analyzes blood components, and detects the number of white blood cells, and a proportion (%) of basophils, a proportion (%) of eosinophils, a proportion (%) of neutrophils, a proportion (%) of lymphocytes, and a proportion (%) of monocytes in the white blood cells.

When the blood cell count detector 21 is a blood cell count receiver, the blood cell count detector 21 receives (acquires) only information of the number of white blood cells, a proportion (%) of basophils, a proportion (%) of eosinophils, a proportion (%) of neutrophils, a proportion (%) of lymphocytes, and a proportion (%) of monocytes in the white blood cells from an existing blood cell count detector, a server, etc.

An example of information that is generated or received by the blood cell count detector 21 will be described with reference to a list of examination results of a blood count of blood components and a white blood cell image of a general subject shown in FIG. 2. In FIG. 2, "WBC" indicates the number of white blood cells in 1 $\mu$l, and in items of a white blood cell image (white blood cell fraction information), "Baso" indicates a proportion (%) of basophils in the white blood cells, "Eosino" indicates a proportion (%) of eosinophils, "Neutro" indicates a proportion (%) of neutrophils, "Lympho" indicates a proportion (%) of lymphocytes, and "Mono" indicates a proportion (%) of monocytes. That is, the blood cell count receiver 212 is required to generate or receive only the number of white blood cells, a proportion (%) of eosinophils, a proportion (%) of neutrophils, a proportion (%) of lymphocytes, and a proportion (%) of monocytes.

The surface antigen CD number detector 22 is composed of, for example, a flow cytometer that detects CD3 antigens, CD56 antigens (and/or CD16 antigens), CD4 antigens, and CD8 antigens, or a receiver for an analysis result of a surface antigen CD number of white blood cells, which receives these pieces of information.

When the surface antigen CD number detector 22 is a flow cytometer, the surface antigen CD number detector 22 may be any unit including a flow cell, a light source, a detector, a signal amplification system part, a conversion system part, etc., and is preferably a unit that does not equip a plurality of photodetectors corresponding to a plurality of types of fluorescence detections.

However, the flow cytometer may be a unit that detects only CD3 antigens, CD56 antigens (and/or CD16 antigens), CD4 antigens, CD8 antigens, etc.

When the surface antigen CD number detector 22 is a CD number analysis result receiver, the surface antigen CD number detector 22 may be a unit that receives (acquires) only information of CD3 antigens, CD56 antigens (and/or CD16 antigens), CD4 antigens, and CD8 antigens from an existing analyzing device, a server, etc.

The storage 5 is composed of hardware (circuits, dedicated logics, etc), software (e.g., a software that operates in a general-purpose computer system or a dedicated machine), etc., and has a function of storing various types of information. The storage 5 stores analysis information obtained by detection or analyzing in the basic information generator 2, the immune cell subgroup detector 3, the immunodynamic change analyzer 4, etc.

The display 6 has a function of displaying analysis information, etc. obtained by detection or analyzing in the basic information generator 2, the immune cell subgroup detector 3, and the immunodynamic change analyzer 4. As the display 6, a general liquid crystal display such as a touch panel or a display panel can be used.

The immune cell subgroup detector 3 detects the total number of helper T cells and the total number of killer T cells in the lymphocyte subgroups (a total number of T cells in the lymphocyte subgroups), the total number of B cells, the total number of NK cells, and the total number of NKT cells based on the number of white blood cells, etc., and an analysis result (analysis information) of the surface antigen CD number of white blood cells (immune cells) acquired at the basic information generator 2. Regarding T cells, the total number of helper T cells and the total number of killer T cells are calculated.

Preferably, the immune cell subgroup detector 3 may be a unit that has an high-speed detection program installed therein, which can detect immune cell subgroup information based on at least the above-mentioned predetermined white blood cell information and its surface antigen CD number analysis information.

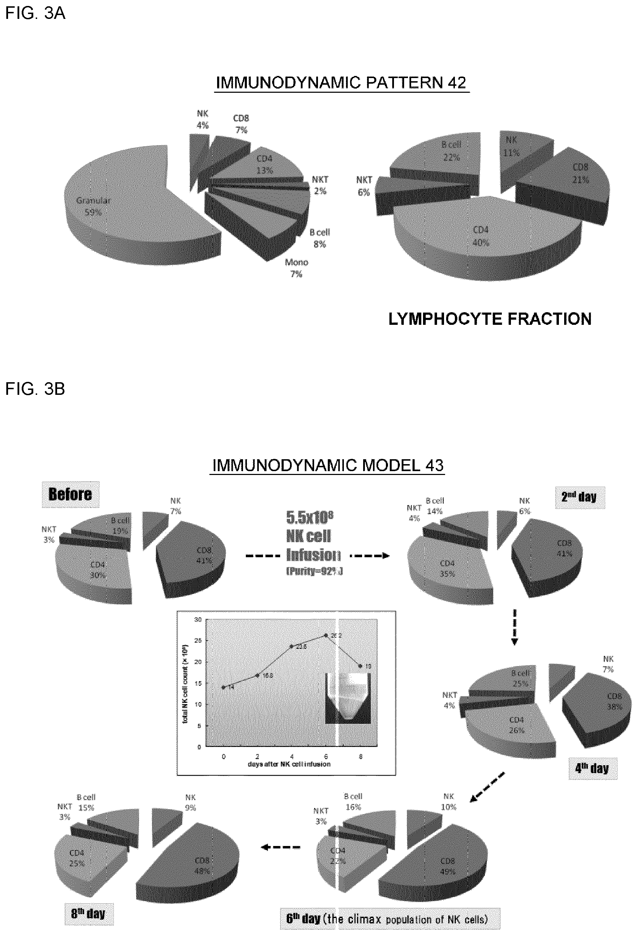

The immunodynamic change analyzer 4 has a function of generating an immunodynamic pattern 42 as shown in FIG. 3A or an immunodynamic model 43 as shown in FIG. 3B based on information detected or obtained by the above-mentioned basic information generator 2 and immune cell subgroup detector 3 (hereinafter referred to as immune cell basic data).

The immune cell basic data includes at least the number of white blood cells, and the numbers and proportions of basophils, eosinophils, neutrophils, lymphocytes, monocytes, CD3 antigens, CD56 antigens (and/or CD16 antigens), CD4 antigens, and CD8 antigens in the white blood cells.

Based on the above-mentioned immune cell basic data of the subject, the immunodynamic change analyzer 4 identifies the immunodynamic pattern of the subject and maps the relation between the subject and the standard value based on the immune cell basic data.

The immunodynamic change analyzing part 4 adaptively provides a prompt for collecting additional data based on the immunodynamic pattern 42 of the subject in consideration of health conditions (pathological conditions). The prompt is generated based on the immunodynamic model 43.

That is, the immunodynamic change analyzer 4 analyzes immunodynamics of the subject more quickly and accurately based on the increase or decrease in the number of white blood cells, granulocytes (eosinophils, basophils, and neutrophils), monocytes, and lymphocytes (helper T cells, killer T cells, B cells, NK cells, and NKT cells), proportions thereof, etc., and displays the analysis result on the display 6.

CD3 is an antigen on the membrane surface of all T cells. CD4 is an antigen on the membrane surface of helper T cells (CD4 positive T cells). CD8 is an antigen on the membrane surface of killer T cells (CD8 positive T cells). CD16 is an antigen on the membrane surface of NK cells. CD56 is an antigen on the membrane surface of NK cells and NKT cells.

Hereinafter, positive may be represented as "+" and negative may be represented as "-." Helper T cells are cells that express a characteristic of "CD3+CD4+," killer T cells are cells that express a characteristic of "CD3+CD8+," B cells are cells that express a characteristic of "CD3-CD56-," NK cells are cells that express a characteristic of "CD3-CD56+," and NKT cells are cells that express a characteristic of "CD3+CD56+."

The CD number analysis result receiver receives at least information of CD3 antigens, CD56 antigens (and/or CD16 antigens), CD4 antigens, and CD8 antigens.

Hereinafter, an example of operations of the multi-parameter automatic blood cell counting device 1 according to the present embodiment will be described with reference to the following Example 1.

Example 1

In a first process, blood is collected from the subject, and the basic information generator 2 analyzes the blood, acquires information of the number of white blood cells, and the proportion (%) of basophils, the proportion (%) of eosinophils, the proportion (%) of neutrophils, the proportion (%) of lymphocytes, and the proportion (%) of monocytes in the white blood cells, and CD classification analysis information of CD3 antigens, CD56 antigens (and/or CD16 antigens), CD4 antigens, and CD8 antigens, and stores the acquired information in the storage 5.

In a second process, the immune cell subgroup detector 3 calculates the total number of the white blood cells (immune cells) in the body of the subject and the total number (number) of each of the white blood cell subgroups (immune cell subgroups) based on the number of the white blood cells and the white blood cell fraction information acquired by the basic information generator 2.

Specifically, the total number of the white blood cells (immune cells) and the white blood cell subgroups (immune cell subgroups: granulocytes (eosinophils, basophils, and neutrophils), monocytes, lymphocytes (helper T cells, killer T cells, B cells, NK cells, and NKT cells)) are detected (calculated) using a high-speed calculation program installed in the immune cell subgroup detector 3. The high-speed calculation program includes at least the following Calculation Formulae (1) to (5). Here, the total number of the white blood cells is calculated by Calculation Formula (1) when the subject is male and calculated by Calculation Formula (2) when the subject is female.

(Math. 1)

The total number of white blood cells

= the number of white cells (/$\mu$l) × 5,000 (blood volume) ml× 1,000 (1,000 $\mu$l=1 ml)   (1)

The total number of white blood cells

= the number of white cells (/$\mu$l) × 4,500 (blood volume) ml× 1,000 (1,000 $\mu$l=1ml)   (2)

The total number of granulocytes

= the total number of white blood cells × granulocytes (basophils, eosinophils, and neutrophils) (%)   (3)

The total number of monocytes = the total number of white blood cells × monocytes (%)   (4)

The total number of lymphocytes = the total number of white blood cells × lymphocytes (%)   (5)

Subsequently, the immune cell subgroup detector 3 detects the total number of T cells in the lymphocyte subgroups based on the above-mentioned detected total number of lymphocytes and the CD classification analysis information acquired by the basic information generator 2.

Specifically, the total number of T cells in the lymphocyte subgroups is detected (calculated) using the high-speed calculation program installed in the immune cell subgroup detector 3 including the following Calculation Formula (6).

Subsequently, the immune cell subgroup detector 3 detects the total number of helper T cells and the total number of killer T cells based on the calculated total number of T cells and the CD classification analysis information acquired by the basic information generator 2.

The total number of helper T cells and the total number of killer T cells are detected (calculated) using the high-speed calculation program installed in the immune cell subgroup detector 3 including the following Calculation Formulae (7) and (8).

Subsequently, the immune cell subgroup detector 3 detects the total number of non-T cells other than T cells based on the detected total number of T cells and the total number of lymphocytes as well as the CD classification analysis information acquired by the basic information generator 2.

The total number of non-T cells is detected (calculated) using the high-speed calculation program installed in the immune cell subgroup detector 3 including the following Calculation Formula (9).

Subsequently, the immune cell subgroup detector 3 detects the total number of B cells, the total number of NK cells, and the total number of NKT cells based on the detected total number of non-T cells and the total number of T cells as well as the CD classification analysis information acquired by the basic information generator 2.

The total number of B cells, the total number of NK cells, and the total number of NKT cells are detected (calculated) using the high-speed calculation program installed in the immune cell subgroup detector 3 including the following Calculation Formulae (10), (11), and (12).

(Math. 2)

The total number of T cells = the total number of lymphocytes × CD3(+) (%)   (6)

The total number of CD4(+) T cells = the total number of T cells × CD4(+) (%)   (7)

The total number of CD8(+) T cells = the total number of T cells × CD8(+) (%)   (8)

The total number of non-T cells = the total number of lymphocytes × (100 − CD3(+))(%)   (9)

The total number of B cells = the total number of non-T cells × ((CD16(-)/CD56(-)) (%)   (10)

The total number of NK cells

= the total number of non-T cells × ((CD16(+)/CD56(+)) + ((CD16(+)/CD56(-)) (%)   (11)

The total number of NKT cells = the total number of T cells × ((CD16(-)/CD56(+)) (%)   (12)

Note that Calculation Formulae (1) to (12) can calculate the number of cells in 1 $\mu$l of each component. The immune cell subgroup detector 3 stores the calculated result data in the storage 5.

In a third process, the immunodynamic change analyzer 4 creates an immunodynamic information of the subject based on the data information (immune cell basic data) detected (calculated) by the above-mentioned basic information generator 2 and the immune cell subgroup detector 3, and the data stored in the storage 5. The immunodynamics in the present application is information related to immune status presented by providing information of the subject based on the numbers and proportions of white blood cells, basophils, eosinophils, neutrophils, lymphocytes, monocytes, CD3 antigens, CD56 antigens (and/or CD16 antigens), CD4 antigens, and CD8 antigens in the white blood cells of the subject detected immediately after blood sampling (within 2 hours after blood sampling).

That is, the immunodynamic change analyzer 4 analyzes the immune status of the subject quickly and accurately based on the numbers and proportions of white blood cells, granulocytes (eosinophils, basophils, and neutrophils), monocytes, and lymphocytes (helper T cells, killer T cells, B cells, NK cells, and NKT cells) detected by the above-mentioned basic information generator 2 and immune cell subgroup detector 3, forms a pattern of the immune status, and displays the immunodynamics of the subject. The immunodynamic change analyzer 4 is composed of hardware (circuits, dedicated logics, etc.), software (e.g., a software that operates in a general-purpose computer system or a dedicated machine), etc.

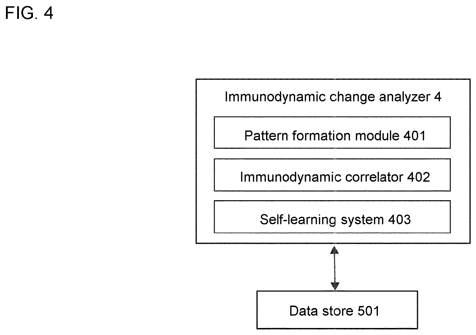

As shown in FIG. 4, the immunodynamic change analyzer 4 specifically includes a pattern formation module 401, an immunodynamic correlator 402, and a self-learning system 403. The immunodynamic change analyzer 4 is connected to one or more data stores 501 and/or storages 5 for storing data (e.g., information, models, feedbacks, subject profiles, etc).

The pattern formation module 401 is an image formation software for forming a pattern of an immune status, and divides the immune status into sections to form an immune status pattern (e,g., an immunodynamic pattern). The self-learning system 403 has a self-learning function of learning in accordance with blood count data and characterizes the correlation between the components of the blood counts.

Pattern Formation Example 1

The functions of the immunodynamic change analyzer 4 will be described based on the pattern formation related to influenza infection.

In Pattern Formation Example 1, first, the self-learning system 403 extracts cell data correlated with influenza virus based on the total number of white blood cells (immune cells) and the white blood cell subgroups (immune cell subgroups) of a certain number of influenza infected persons (hereinafter referred to as infected persons) selected at random, which are stored in the storage 5 (or the data store 501), and stores the extracted data in the storage 5 (or the data store 501). Here, the cell data extracted by the self-learning system 403 includes the numbers of total granulocytes, total lymphoid cells, CD4 antigens, CD8 antigens, NK cells, NKT cells, and B cells shown in Table 1.

[Table 1]

| | 21-year -old male | 37-year -old male | 31-year -old male | 47-year -old female | 32-year -old male | 35-year -old male | 48-year -old female | 25-year -old female | 48-year -old male | 65-year -old male |
|---|---|---|---|---|---|---|---|---|---|---|
| Total granulocytes | 4401 | 4073 | 4068 | 2026 | 2545 | 2347 | 1908 | 2754 | 2850 | 7250 |
| Total lymphoid cells | 377 | 631 | 291 | 169 | 269 | 274 | 581 | 325 | 351 | 921 |
| CD4 | 79 | 82 | 54 | 40 | 40 | 45 | 108 | 55 | 46 | 97 |
| CD8 | 97 | 64 | 40 | 33 | 84 | 57 | 97 | 33 | 40 | 178 |
| NK | 31 | 136 | 27 | 13 | 43 | 56 | 87 | 23 | 29 | 146 |
| NKT | 18 | 14 | 5 | 6.6 | 15 | 15 | 10 | 6 | 10 | 27 |
| B | 90 | 202 | 110 | 45 | 46 | 78 | 166 | 123 | 121 | 268 |

The self-learning system 403 determines a pattern range (influenza infection pattern and pseudo-influenza infection pattern) indicating the characteristic of influenza infection based on the numbers of total granulocytes, total lymphoid cells, CD4 antigens, CD8 antigens, NK cells, NKT cells, and B cells stored in the storage 5 (or the data store 501), and stores the determined range in the storage 5 (or the data store 501). The data in Table 1 is calculated by the immune cell subgroup detector 3 based on the number of the white blood cells and the white blood cell fraction information of the infected persons acquired by the above-mentioned basic information generator 2, and corresponds to infected immune cell basic data of the infected persons extracted by the self-learning system 403.

The self-learning system 403 calculates the total number of the number of CD4 antigens, the number of CD8 antigens, the number of NK cells, the number of NKT cells, and the number of B cells of the infected persons based on the infected immune cell basic data of the infected persons shown in Table 1, calculates the proportions of the number of CD4 antigens, the number of CD8 antigens, the number of NK cells, the number of NKT cells, and the number of B cells shown in the following Table 2, and stores the results in the storage 5 (or the data store 501).

[Table 2]

| | 21-year-old male | 37-year-old male | 31-year-old male | 47-year-old female | 32-year-old male | 35-year-old male | 48-year-old female | 25-year-old female | 48-year-old male | 65-year-old male | Average value |
|---|---|---|---|---|---|---|---|---|---|---|---|
| CD4 | 25% | 16% | 23% | 29% | 18% | 18% | 23% | 23% | 19% | 14% | 21% |
| CD8 | 31% | 13% | 17% | 24% | 37% | 23% | 21% | 14% | 16% | 25% | 22% |
| NK | 10% | 27% | 11% | 9% | 19% | 22% | 19% | 10% | 12% | 20% | 16% |
| NKT | 6% | 3% | 2% | 5% | 7% | 6% | 2% | 2% | 4% | 4% | 4% |
| B | 28% | 41% | 47% | 33% | 20% | 31% | 35% | 51% | 49% | 37% | 37% |
| C04/B | 0.88 | 0.41 | 0.49 | 0.89 | 0.87 | 0.58 | 0.65 | 0.45 | 0.38 | 0.36 | 0.59 |
| **Total lymphoid cells /total granulocytes** | 0.09 | 0.15 | 0.07 | 0.08 | 0.11 | 0.12 | 0.30 | 0.12 | 0.12 | 0.13 | 0.13 |
| **Body temperature (°C)** | 39. 4 | 38.6 | 38. 8 | 38.2 | 40 | 38.8 | 38. 8 | 39 | 38.5 | 38. 4 | 38.8 |
| **Pulse rate (/min)** | 97 | 122 | 100 | 88 | 118 | 125 | 115 | 102 | 110 | 130 | 111 |

In addition, the self-learning system 403 simultaneously shows reference data determined to be related to the characteristic of influenza infection, for example, the body temperature and the pulse rate as shown in Table 2, when the pattern range indicating the characteristic of the influenza infection is determined.

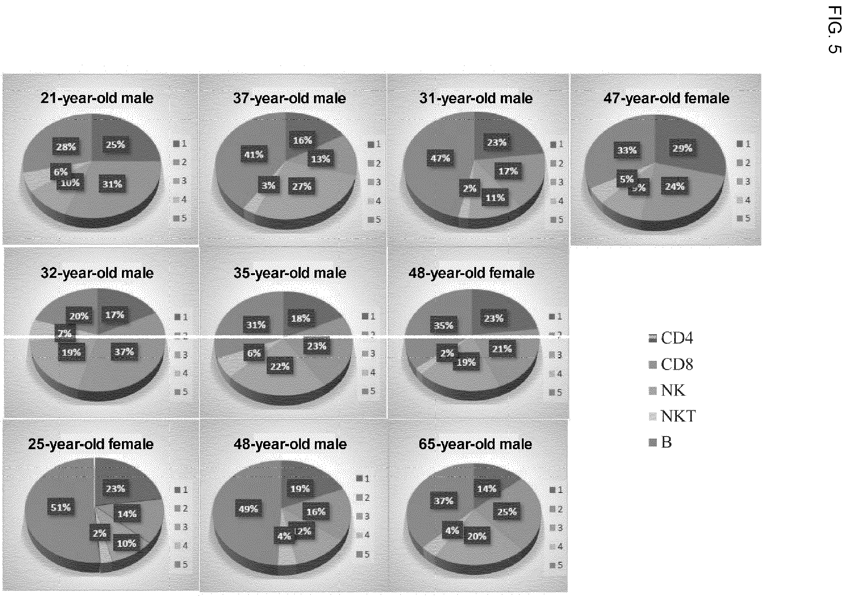

Next, the pattern formation module 401 forms an image of the proportions of the number of CD4 antigens, the number of CD8 antigens, the number of NK cells, the number of NKT cells, and the number of B cells of the infected persons shown in the above-mentioned Table 2, which are stored in the storage 5 (or the data store 501), and displays this image on the display 6. FIG. 5 shows diagrams of the proportions of the number of CD4 antigens, the number of CD8 antigens, the number of NK cells, the number of NKT cells, and the number of B cells of infected persons displayed on a display.

In addition, the self-learning system 403 calculates the ratio of the number of CD4 antigens to the number of B antigens (CD4/B), and the ratio of total lymphoid cells to total granules (total lymphoid cells/total granules) of each infected person, and stores the results in the storage 5 (or the data store 501). Moreover, the self-learning system 403 calculates respective average values (referred to as comparative influenza infection pattern standard data) of the proportion of CD4 antigens, the proportion of CD8 antigens, the proportion of NK cells, the proportion of NKT cells, and the proportion of B cells of the infected persons shown in Table 2, and stores the results in the storage 5 (or the data store 501).

The pattern formation module 401 can display information for determining whether or not the subject is infected with influenza on the display 6 using the comparative influenza infection pattern standard data stored in the storage 5.

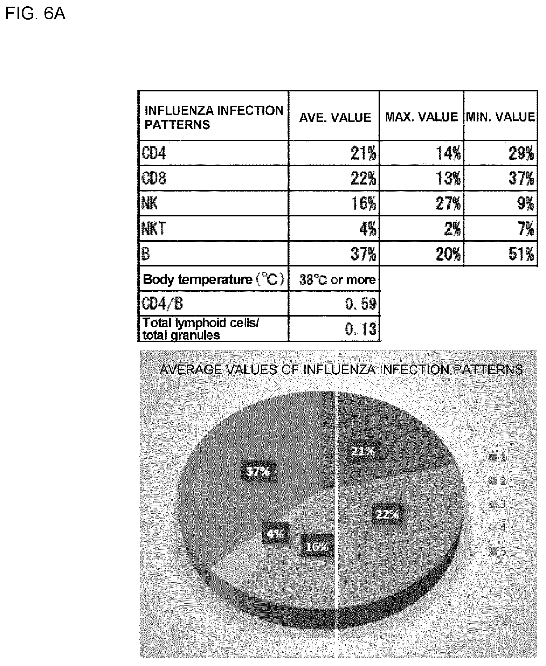

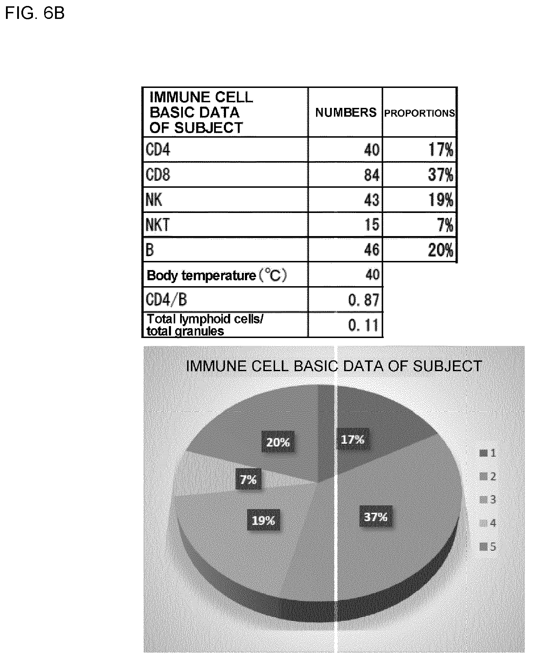

That is, in the multi-parameter automatic blood cell counting device 1 according to the present embodiment, the self-learning system 403 compares immune cell basic data of the subject with various types of data stored in the storage 5, selects immune cell data (hereinafter referred to as comparative immune cell data) close to the immune cell basic data of the subject, and displays the results on the display 6 (see FIGS. 6A and 6B). Therefore, the staff can easily determine whether or not the subject is infected with influenza virus by referring to the immune cell basic data of the subject and the comparative immune cell data displayed on the display 6, and can prevent the spread of infection at an early stage.

Hereinafter, a function of displaying infectious disease determination information of the multi-parameter automatic blood cell counting device 1 according to the present embodiment will be described using clinical cases of actual influenza infection patterns.

Determination Example 1

First, in a medical field in which the multi-parameter automatic blood cell counting device 1 according to the present embodiment is provided, blood of a subject is collected, the basic information generator 2 is used to acquire the number of the white blood cells of the subject, the white blood cell image (white blood cell fraction information), and the analysis result of the surface antigen CD number of the white blood cells (immune cells), and the acquired results are stored in the storage 5.

Next, the immune cell subgroup detector 3 calculates the total number of the white blood cells (immune cells) in the body of the subject and the total number (number) of each of the white blood cell subgroups (immune cell subgroups) based on the number of the white blood cells and the white blood cell fraction information acquired by the basic information generator 2, and stores the acquired results in the storage 5.

The pattern formation module 401 creates an image of the numbers and the proportions of total granulocytes, total lymphoid cells, CD4 antigens, CD8 antigens, NK cells, NKT cells, and B cells (hereinafter referred to as immune cell basic data of the subject) from the total number of the white blood cells (immune cells) and the white blood cell subgroups (immune cell subgroups) of the above-mentioned subject, and displays the image on the display 6.

Subsequently, the pattern formation module 401 instructs the self-learning system 403 to select comparative immune cell data similar to the immune cell basic data of the subject from the immune cell data group stored in the storage 5 (or the data store 501) (selection operation).

When the self-learning system 403 determines and selects, from the immune cell data group stored in the storage 5 (or the data store 501), the comparative immune cell data similar to the immune cell basic data of the above-mentioned subject, the self-learning system 403 reports, to the pattern formation module 401, that the selection operation is completed. Here, the comparative immune cell data selected by the self-learning system 403 is comparative influenza infection pattern standard data.

After the pattern formation module 401 receives, from the self-learning system 403, the report that the selection operation is completed, it displays the comparative influenza infection pattern standard data on the display 6.

As a result, the pattern formation module 401 displays simultaneously the immune cell basic data of the subject and the comparative influenza infection pattern standard data on the display part 6. In addition, when the pattern formation module 401 determines that the immune cell basic data of the subject is within the range of the comparative influenza infection pattern standard data, it displays an influenza infection pattern (positive). When the pattern formation module 401 determines that the immune cell basic data of the subject is not within the range of the comparative influenza infection pattern standard data, the pattern formation module 401 displays that it is not an influenza infection pattern (negative).

FIG. 6A is a diagram showing immune cell basic data of the subject that is displayed on the display 6 by the pattern formation module 401. FIG. 6B is a diagram showing comparative influenza infection pattern standard data that is displayed on the display 6 by the pattern formation module 401.

Medical staff can determine the infection status of the subject simply and quickly by referring to the immune cell basic data of the subject and the comparative influenza infection pattern standard data as shown in FIG. 6A and FIG. 6B above, and can detect infected persons at an early stage and effectively prevent the spread of infection.

The pattern formation module 401 in the present embodiment is not limited to displaying the above-mentioned immune cell basic data of the subject and the comparative immune cell data. The pattern formation module 401 in the present embodiment has a function of calculating the proportions of the number of NK cells, the number of helper T cells, the number of killer T cells, the number of B cells, and the number of NKT cells of the subject, forming a graph, and forming pattern classification therefrom.

In the pattern classification, for example, the immunodynamic (normal or abnormal) pattern of the immune status is identified based on the number and the proportions of cells in respective components of the white blood cell subgroups, the number and the proportions of cells of monocytes and lymphocyte subgroups, and the number and the proportion of cells of only the lymphocyte subgroups.

Hereinafter, an operation of the pattern formation module 401 forming pattern classification will be described using an output example (normal immune status pattern I) of the immune status of healthy persons with no disease (hereinafter referred to as "healthy persons"), an output example (abnormal condition I) of the immune status of patients with gastric cancer, an output example (abnormal condition II) of patients with rheumatoid arthritis, and an output example (abnormal condition III) of the immune status with patients with atopic dermatitis.

Output Example 1

First, blood of patients with gastric cancer (subject) is collected, and the basic information generator 2 and the immune cell subgroup detector 3 acquire immune cell basic data of the patients with gastric cancer.

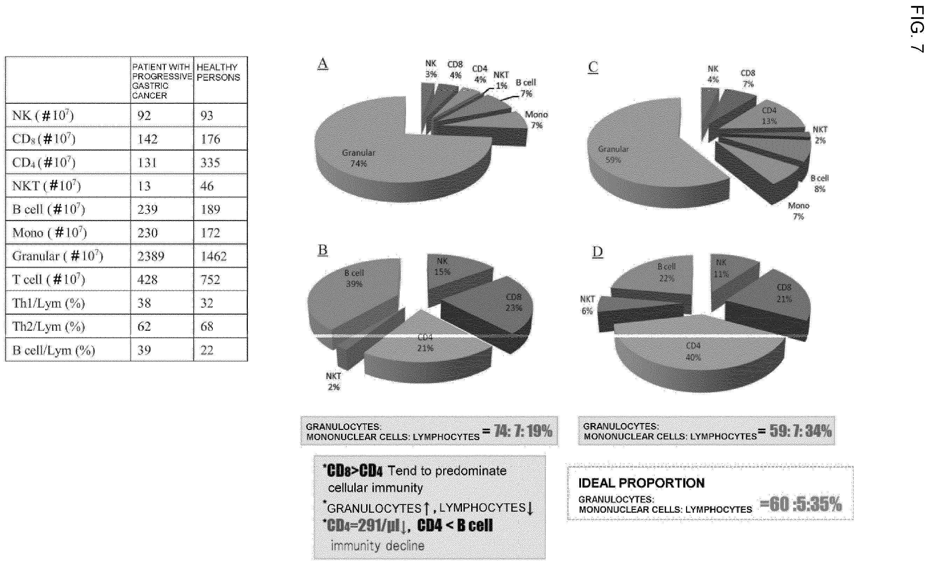

Next, the pattern formation module 401 generates pie charts of the immune status of the patients with gastric cancer as shown in (A) and (B) in FIG. 7 based on the immune cell basic data of the patients with gastric cancer, and outputs the results on display 6. The multi-parameter automatic blood cell counting device 1 according to the present embodiment can immediately display the immune status of the patients with gastric cancer.

In order to check the immune status of the patients with gastric cancer, the pattern formation module 401 displays comparative information with respect to the immune status of healthy persons. Specifically, the pattern formation module 401 acquires data of the immune status of healthy persons suitable for comparison to the patients with gastric cancer from the storage 5, generates pie charts of the immune status of healthy persons as shown in (C) and (D) in FIG. 7, and displays the results on the display 6. In this output example, the pattern formation module 401 simultaneously displays the pie charts of the immune status of healthy persons as shown in (C) and (D) in FIG. 7, and the pie charts of the immune status of the patients with gastric cancer as shown in (A) and (B) in FIG. 7.

(A) in FIG. 7 is a pie chart showing the proportions of components of the white blood cell subgroups of patients with gastric cancer. (B) in FIG. 7 is a pie chart showing the proportion of only the lymphocyte subgroups of patients with gastric cancer. (C) in FIG. 7 is a pie chart showing the proportions of components of the white blood cell subgroups of healthy persons. (D) in FIG. 7 is a pie chart showing the proportion of only the lymphocyte subgroups of healthy persons.

As shown in (A) and (B) in FIG. 7, the number of CD4 cells of the patients with gastric cancer is 1.31 billion, granulocytes : mononuclear cells : lymphocytes = 74 : 7 : 19%, and sympathetic nerve dominance and granulocyte-proliferative lymphocyte decrease are shown. The number of cells of the immune phase central lymphocyte subgroups $\approx$ 262 cells/$\mu$l (< 300 cells/$\mu$l), and the proportion value of CD4 is smaller than the proportion value of B cells and the proportion value of CD8, and suggests immune-decline. In addition, the proportion of TH1 cell-mediated immunity group (NK group + CD8 group) > TH2-mediated immunity group (NKT group + B group) tends to predominate cellular immunity. In this case, the pattern formation module 401 identifies the immune status of the patients with gastric cancer as an "immune-decline pattern."

In addition, the pattern formation module 401 forms a pattern based on the following immunity evaluation reference data A) to C).

A) Self-immunity Healthy-degree (health level of self-immunity)

- Number of cells of CD4 Helper-T cell subgroups (=helper T cell immunity center)/$\mu$L/ml

- CD4 > 600/$\mu$l $\rightarrow$ normal Immunity (normal)

- CD4 600 to 400/$\mu$l $\rightarrow$ Immune-decline

- CD4 400 to 200/μl → Immune-impaired

- CD4 < 200/μl > → Immune-crisis

B) Body direction of immunity

- Th1 cell-mediated immunity = NK + CD8 (killer-T)

- Th2 humoral immunity = NKT + B cell

- CD4 > CD8 > B cell direct proportion (≈ lymphatic subgroup proportional pattern)

C) Body immune autonomic nerve balance

Granulocytes: mononuclear cells : lymphocytes = 60 : 5 : 35% (average value for normal persons)
→ sympathetic nerves (granulocytes > lymphocytes%)/parasympathetic nerve dominance (lymphocytes > granulocytes%)

D) Compensatory change in body immune cell group (based on monitoring data from beginning due to constant change)

· Other patterns that change

In this example, the above-mentioned immunity evaluation reference data A) to C) are stored in the data store 501 (or the storage 5) in advance.

Meanwhile, as shown in (C) and (D) in FIG. 7, the number of CD4 cells of healthy persons is 3.35 billion, and granulocytes : mononuclear cells : lymphocytes = 59 : 7 : 34%. The average value for normal persons is granulocytes : mononuclear cells : lymphocytes = 60 : 5 : 35%.

In this case, the multi-parameter automatic blood cell counting device 1 of the present embodiment shows that the immune status of the patients with gastric cancer is an "immune-decline pattern," displays a characteristic value indicating a decline in immunity (for example, the numerical value on the left side in FIG. 7) in comparison with the above-mentioned immune status of the healthy persons, and stores the results in the storage 5.

Therefore, using the multi-parameter automatic blood cell counting device 1 of the present embodiment, the medical staff can check the immune status of the above-mentioned examinee in a timely manner, acquire the dynamic state of immunity of the examinee, and accurately ascertain the immune status. In addition, the multi-parameter automatic blood cell counting device 1 of Example 1 can be composed of hardware (circuits, dedicated logics, etc.) and software (e.g., a software that operates in a general-purpose computer system or a dedicated machine) required for analyzing the number of white blood cells, the white blood cell image (white blood cell fraction information), and the surface antigen CD number of certain white blood cells (immune cells). As a result, the multi-parameter automatic blood cell counting device 1 of the present embodiment is made compact. Moreover, data related to immune status that was conventionally acquired by at least a combination of a number of devices such as a blood cell count detection device, a flow cytometer, and a PCR can be acquired immediately using only the multi-parameter automatic blood cell counting device 1 of the present embodiment.

In addition, when the medical staff uses the multi-parameter automatic blood cell counting device 1 of the present embodiment, it is possible to visually identify data related to the immunodynamics of the examinee and the pattern of the immune status, perform identification quickly and accurately, and shorten the examination time without performing separate calculations as shown in Patent Document 1 in order to acquire immunodynamics.

Output Example 2

Output Example 2 shows an example in which the immune status belonging to the Th2 humoral immune dominant tendency pattern is output. In consideration of the characteristics of the Th2 humoral immune dominant tendency pattern, an example in which subjects are patients with rheumatoid arthritis and patients with atopic dermatitis is described.

As in Output Example 1, first, blood of the subject is collected, and the basic information generator 2 and the immune cell subgroup detector 3 acquire immune cell basic data 41 of the subject.

Next, the pattern formation module 401 generates pie charts of the immune status of patients with rheumatoid arthritis and the immune status of patients with atopic dermatitis as shown in FIG. 8 based on the immune cell basic data 41 of the subjects. (A) in FIG. 8 is a pie chart showing the proportions of components of the white blood cell subgroups of patients with rheumatoid arthritis. (B) in FIG. 8 is a pie chart showing the proportions of the lymphocyte subgroups and the single cells of patients with rheumatoid arthritis. (C) in FIG. 8 is a pie chart showing the proportion of only the lymphocyte subgroups of patients with rheumatoid arthritis. (D) in FIG. 8 is a pie chart showing the proportions of components of the white blood cell subgroups of patients with atopic dermatitis. (E) in FIG. 8 is a pie chart showing the proportions of the lymphocyte subgroups and the single cells of patients with atopic dermatitis. (F) in FIG. 8 is a pie chart showing the proportion of only the lymphocyte subgroups of patients with atopic dermatitis.

As shown in (A) and (C) in FIG. 8, the number of CD4 cells of an 82-year-old female patient with rheumatoid arthritis is 870 million, the number of CD8 cells is 330 million, and granulocytes : mononuclear cells : lymphocytes = 79 : 5 : 16%. The proportion value of CD4 of patients with rheumatoid arthritis is smaller than the proportion value of B cells (inverse proportional phenomenon of the lymphocyte subgroups) but is larger than the proportion value of CD8, and since the Th2 humoral immune group proportion (NKT group + B group) > Th1 cell-mediated immunity (NK group + CD8 group), a Th2 humoral immune dominant tendency pattern is shown. In this case, the multi-parameter automatic blood cell counting device 1 of the present embodiment determines that the immune status of the patients with rheumatoid arthritis is a "Th2 humoral immune dominant tendency pattern", and displays the characteristic of the immune status in comparison with the immune status of healthy persons.

As shown in (D) and (F) in FIG. 8, the number of CD4 cells of patients with atopic dermatitis is 1.93 billion, the number of CD8 cells is 1.99 billion, and granulocytes : mononuclear cells : lymphocytes = 65 : 5 : 30%. In addition, the proportion value of CD4 of patients with atopic dermatitis is smaller than the proportion value of B cells (inverse proportional phenomenon of the lymphocyte subgroups), and is almost equal to the proportion value of CD8. As in the above-mentioned patients with rheumatoid arthritis, since the Th2 humoral immune group proportion (NKT group + B group) > Th1 cell-mediated immunity (NK group + CD8 group), a Th2 humoral immune dominant tendency pattern is shown. In this case, the multi-parameter automatic blood cell counting device 1 of the present embodiment shows that the immune status of the patients with atopic dermatitis is a "Th2 humoral immune dominant tendency pattern", and displays the characteristic of the immune status in comparison with the immune status of healthy persons.

Output Example 3

Output Example 3 describes an example in which, in order to accurately determine the immune status and the pathological conditions and in order to compare with other pathological condition patterns, a pathological condition pattern (comparative state pattern) different from the immune status pattern of the subject is simultaneously displayed for comparison. Since the immune cell basic data 41 of the subject is acquired by the same method as in Output Example 1, descriptions thereof will be omitted.

The pattern formation module 401 generates pie charts of the immune status of patients with esophageal candida and the immune status of patients with psoriasis as shown in FIG. 9 based on the immune cell basic data 41 of the subjects. (A) in FIG. 9 is a pie chart showing proportions of components of the white blood cell subgroups of patients with esophageal candida. (B) in FIG. 9 is a pie chart showing the proportion of only the lymphocyte subgroups of patients with esophageal candida. (C) in FIG. 9 is a pie chart showing the proportions of components of the white blood cell subgroups of patients with psoriasis. (D) in FIG. 9 is a pie chart showing the proportion of only the lymphocyte subgroups of patients with psoriasis.

As shown in (A) and (B) in FIG. 9, the number of CD4 cells of patients with esophageal candida is 1.32 billion, the number of CD8 cells is 1.73 billion, and granulocytes : mononuclear cells : lymphocytes = 67 : 6 : 27%. In addition, the number of CD4 cells of patients with esophageal candida indicates the immune-decline state of 264 cells/$\mu$l (< 400 cells/$\mu$l), and the proportion value is smaller than the proportion value of B cells and the proportion value of CD8, and this suggests an inverse proportional phenomenon of the lymphocyte subgroups. Since the Th1 cell-mediated immune group proportion (NK group + CD8 group) > Th2 humoral immunity (NKT group + B group), a cell-mediated immunity dominant tendency is shown. In this case, the multi-parameter automatic blood cell counting device 1 of the present embodiment shows that the immune status of the patients with esophageal candida is a cell-mediated immunity dominant tendency pattern, and displays the characteristic of the immune status in comparison with the immune status of healthy persons.

Meanwhile, the immune status of patients with psoriasis shown in (C) and (D) in FIG. 9 has a value that is significantly different from that of the immune status of patients with esophageal candida. Granulocytes : mononuclear cells : lymphocytes of patients with psoriasis = 67 : 8 : 25%, which are values almost the same as those of the above-mentioned patients with esophageal candida, and CD4 of patients with psoriasis indicates an immunostimulatory state with 4.16 billion and 832 cells/$\mu$l (> 600 cells/$\mu$l), which is larger than 760 million for CD8. In addition, the proportion value of CD4 of patients with psoriasis is much larger than the proportion value of B cells and the proportion value of CD8, and the proportion of NKT cells is almost zero, which suggests a possibility of a Th17 cell-mediated immunity dominant tendency rather than the Th2 humoral immune dominant tendency pattern. In this case, the multi-parameter automatic blood cell counting device 1 of the present embodiment suggests that the immune status of the patients with psoriasis is a "Th17 cell-mediated immunity dominant tendency pattern," and displays the characteristic of the immune status in comparison of the immune status of healthy persons.

The comparative state pattern in Output Example 3 is provided by the self-learning system 403 of the present application.

The self-learning system 403 can select one or more comparative state patterns having the best correlation with the immune status of the subject by statistical calculation (e.g., mathematical optimization calculation) or selection by a clinical staff based on data stored in the data store 501. For example, numerical analysis software can be used for mathematical optimization calculation. As an example, general numerical analysis software SciPy may be used.

For example, the self-learning system 403 is connected to at least the data store 501 in which immunodynamic analysis reference data is stored. The immunodynamic analysis reference data is only an example as reference information for analyzing and defining the change in immunodynamics, and is beneficial information for clinical staff when analyzing and defining correlation statistical data of the change in immunodynamics.

Reference information data for analyzing and defining the change in immunodynamics may be information supplemented in a timely manner based on information of medical clinical fields and information of specialized magazines, etc.

In addition, the clinical staff can additionally supplement the reference information data for analyzing and defining the change in immunodynamics, based on their own experience, to create data specialized in the therapeutic areas of expertise.

Regarding the statistical calculation of the self-learning system 403, if a comparative state pattern having the best correlation with the immune status of the subject can be selected, the following simple calculation method may be used for selection.

In Calculation Example 1, multiple regression analysis is performed using the number of NK cells as a dependent variable, and the numbers of helper T cells, killer T cells, B cells, and NKT cells as independent variables.

The partial regression coefficients of the numbers of helper T cells, killer T cells, B cells, and NKT cells are obtained and a significance difference test is performed and the results are displayed.

Next, a multiple correlation coefficient is obtained, a significance difference test is performed, and the results are displayed.

Contribution rates of the numbers of helper T cells, killer T cells, B cells, and NKT cells are obtained and displayed.

The correlation between the number of NK cells and the numbers of helper T cells, killer T cells, B cells, and NKT cells is obtained and displayed.

Regarding an example of correlation involved in activation of cell-mediated immunity, a total sum of NK cells and killer T cells and the increase or decrease thereof, etc. are analyzed to compare the Th1 cell-mediated immune status in which Th1 cells are involved with the immune status of the subject, thereby selecting the most suitable data from the statistical data group. Th1 cells mainly produce IFN-$\gamma$, and are involved in activation of cell-mediated immunity, etc.

In Calculation Example 2, based on the correlation between the numbers of NKT cells and NK cells, helper T cells, killer T cells, and B cells, accurate correlation information of the humoral immune status, etc. is obtained and compared with the immune status of the subject, thereby selecting the most suitable data from the statistical data group.

The multiple regression analysis of Calculation Example 2 is performed by the same method as in the above-mentioned Calculation Example 1. Moreover, the correlation between the numbers of NKT cells and NK cells, helper T cells, killer T cells, and B cells is obtained and compared with the immune status of the subject, thereby selecting the most suitable data from the statistical data group.

Regarding an example of a correlation involved in the humoral immune status, a total sum of NKT cells and B cells and the increase or decrease thereof, etc. are analyzed to make it possible to ascertain the Th2 humoral immune status in which Th2 cells are involved. Th2 cells mainly produce interleukin-4 (IL-4) and are involved in humoral immunity.

The above-mentioned Calculation Example 1 and Calculation Example 2 are only examples in which a correlation for analyzing the change in immunodynamics is derived. In addition to the correlation method indicating immunodynamics in the above-mentioned Calculation Example 1 and Calculation Example 2, the multi-parameter automatic blood cell counting device of the present embodiment further includes a method that presents significant numerical values indicating immunodynamics based on optimization calculation, etc. That is, this method provides a comparative state pattern for statistical analysis related to the correlation of the immune cell subgroups and change in immunodynamics acquired based on the information acquired using the immune cell basic data 41 of the subject, and if it can be immediately applied to the immune status of the subject, it can be used for the multi-parameter automatic blood cell counting device of the present embodiment. In addition, it can be applied to creation of an image of new information analysis by joint analysis of analysis data of the multi-parameter automatic blood cell counting device of the present embodiment and measurement data in other fields. For example, it is possible to help investigate the Th17 mucosal immunity field by joint analysis of analysis data of the multi-parameter automatic blood cell counting device of the present embodiment and bacterial flora analysis data in intestinal feces.

Next, operations of the immunodynamic correlator 402 will be described based on Output Example 4.

The immunodynamic correlator 402 displays information related to the immunodynamic correlation of the subject with respect to the immune status pattern of the subject formed by the pattern formation module 401. That is, the change in the immune status (change in immunodynamics) of the subject by administering related cells is made into sequential images and displayed, and a tendency change amount and a reference required dose are displayed.

Output Example 4

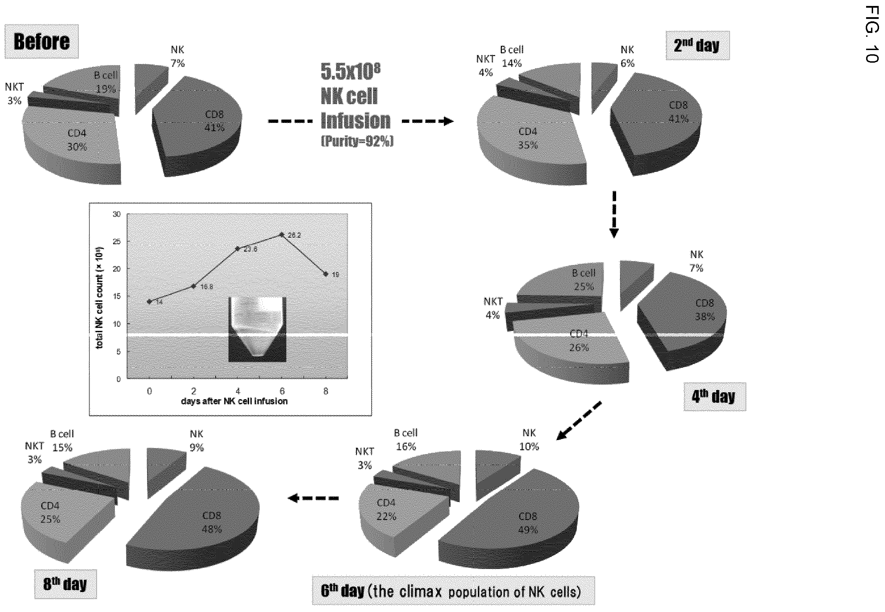

In Output Example 4, NK cells (self-cultured cells) are administered to a subject (subject 1) having an NK cell decrease pattern, and the change in the immune status (change in the immunodynamics) is made into sequential images and displayed, thereby forming an immunodynamic model 43. FIG. 10 is a diagram showing the immunodynamic model 43 formed by the immunodynamic correlator 402 according to the present embodiment.

As shown in FIG. 10, the immunodynamic correlator 402 creates images of the effect of administration of NK cells using physical methods such as infusion or injection, and displays these images. The medical staff and the subject 1 can directly, visually, and immediately check the immunodynamics of the subject. In addition, in Output Example 4, NK cells are administered to a subject having an NK cell decrease pattern, and the immunodynamic correlator 402 forms an immunodynamic model 43 indicating the change in the immune status of the subject for a certain period. Specifically, the results of the following operations are imaged and displayed.

First, blood of the subject is collected before NK cells are injected and the results of analysis (the immune cell basic data 41 before administration) are stored in the storage 5.

Next, self-cultured NK cells ($5.5 \times 108$ NK cells) having a purity of 92% are injected into the subject, and blood is collected again two days later, and analysis is performed using the basic information generator 2 and the immune cell subgroup detector 3, thereby generating an immune cell basic data 41 after administration.

The pattern formation module 401 immediately identifies each of an NK cell decrease pattern and a first NK cell compensation pattern 1 which are the immunodynamic pattern 42 of the subject based on the immune cell basic data 41 before administration and the immune cell basic data 41 after administration.

The immunodynamic correlator 402 displays a graph showing the increase or decrease in the number of NK cells in the body of the subject 1 based on the NK cell decrease pattern and the first NK cell compensation pattern 1 of the subject 1.

As shown in FIG. 10, when autologous NK cells are injected once by infusion, amplification of Th1 cell-mediated immunity is observed. In addition, when NK cells are injected by infusion, amplification of NK cells in the body is also directly observed.

That is, when a drug contributing to NK cells is injected, the medical staff can ascertain quickly and easily information of effects and changes thereof based on the multi-parameter automatic blood cell counting device 1 of the present embodiment, which results in reducing the burden on the patient, and early detection and early treatment.

In addition, the immunodynamic correlator 402 forms a graph that shows the proportion between the dose of NK cells and the increased amount of NK cells in the body of the subject 1, and calculates and displays the digestibility 1 of the dose 1 of NK cells.

In addition, the immunodynamic correlator 402 uses the self-learning system 403 to select an optimal data 1 from the data store 501 using the NK cell decrease pattern 1 and the first NK cell compensation pattern 1 of the above-mentioned subject 1 as samples. The optimal data 1 is data including information of immune status patterns that are the same as or similar to the NK cell decrease pattern 1 and the first NK cell compensation pattern 1 (the NK cell decrease pattern and the first NK cell compensation pattern, a second NK cell compensation pattern, a third NK cell compensation pattern, a fourth NK cell compensation patteren, and so on).

Then, the immunodynamic correlator 402 displays the NK cell decrease pattern 1 and the first NK cell compensation pattern 1, the second NK cell compensation pattern 1, the third NK cell compensation pattern 1, and the fourth NK cell compensation pattern 1 of the subject 1 from the optimal data 1. In addition, the immunodynamic correlator 402 displays the dose and digestibility for the second NK cell compensation pattern 1 of the optimal data 1 and displays an estimated dose 2. The estimated dose 2 is a required dose of NK cells for forming the ideal second NK cell compensation pattern of the subject 1. The estimated dose 2 is obtained by a calculation method of the self-learning system 403 based on the dose 1 and the digestibility 1 of the above-mentioned subject and the dose and the digestibility of the optimal data 1.

In addition, the immunodynamic correlators 402 generates and displays an immunodynamic model 1 of the subject 1 based on the NK cell decrease pattern 1 and the first NK cell compensation pattern 1 for the subject 1, and the optimal data 1. As shown in FIG. 10, the immunodynamic model 1 of the subject 1 is a model in which the change in immunodynamics from the NK cell decrease pattern 1 to the fourth NK cell compensation pattern 4 is predicted.

The immunodynamic model 1 of the subject 1 allows the medical staff to immediately and dynamically ascertain the immune status of the subject 1, and immediately determine what measures to take. The immunodynamic model 1 allows the subject 1 to directly and immediately check his or her own immunodynamics visually, and to accurately ascertain his or her own immunity information.

FIG. 11 shows examples in which the change in immunodynamics of NK cells is imaged and displayed for healthy persons.

(A) in FIG. 11 is a pie chart showing the proportions of components of the white blood cell subgroups of the subject before NK cells are administered and a calculation example of the number of white blood cell subgroups calculated based on these proportions is shown on the left side of the pie chart. (B) in FIG. 11 is a pie chart showing the proportions of monocytes and lymphocyte subgroups of the subject before NK cells are administered. (C) in FIG. 11 is a pie chart showing the proportion of only the lymphocyte subgroups of the subject before NK cells are administered.

(D) in FIG. 11 is a pie chart showing the proportions of components of the white blood cell subgroups of the subject after NK cells are administered and a calculation example of the number of white blood cell subgroups calculated based on these proportions is shown on the right side of the pie chart. (E) in FIG. 11 is a pie chart showing the proportions of monocytes and lymphocyte subgroups of the subject after NK cells are administered. (F) in FIG. 11 is a pie chart showing the proportion of only the lymphocyte subgroups of the subject after NK cells are administered.

As shown in FIG. 11, even after autologous NK cells are injected into a healthy person three times, there is no Th1 immunity amplification phenomenon (CD8↑), and instead, an increasing tendency in the number of immune central lymphocytes CD4 (helper T cells) can be confirmed.

That is, the multi-parameter automatic blood cell counting device 1 of the present embodiment can examine the immune status of healthy persons and immediately confirm compensation of the immune status in a dynamic state, and thus is beneficial for protecting health.

In addition, it is possible to immediately determine the immune status of influenza patients based on the multi-parameter automatic blood cell counting device of the present embodiment.

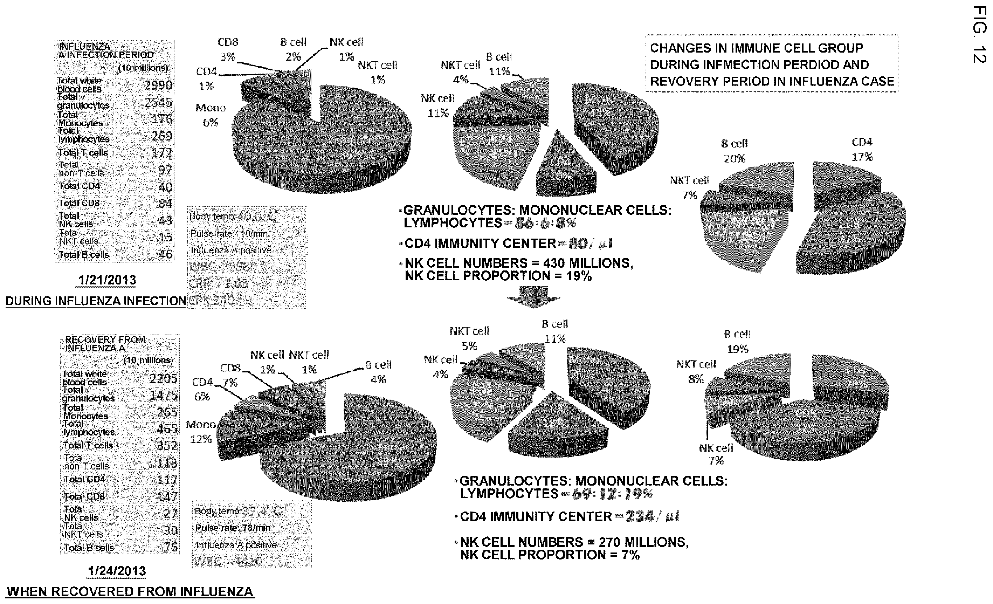

Information displayed by the immunodynamic correlator 402 is not limited to Output Example 4, but can be used to display an immunity correlation from an influenza pattern 1 to a recovery pattern 1 as shown in FIG. 12. The recovery pattern 1 shows an immune status when the health condition of the influenza patient is recovered by treatment.

As shown in FIG. 12, in acute viral infection such as an influenza infectious disease, at the early stage, a maintenance tendency of NK cells (slow tendency of increase to decrease) and a significant decrease of CD4 cells are observed. On the other hand, during a recovery period, a decrease in the number of NK cells and an increase in the number of CD4 cells are observed. An amplification phenomenon (Booster role) of Th1 cell-mediated immunity due to the increase in the number of NK cells at the early stage of infection suggests an early initiation of natural immunity in which infected cells are removed. An increase in the number of CD4 cells during a recovery period is assumed to be caused by initiation of acquired immunity. Therefore, in the case of an acute viral infectious disease, it is suggested that a rapid action of natural immunity by maintaining the number of NK cells is a key for early recovery from infection and avoiding aggravation.

Internal immunity against infection takes about one to two weeks from when natural immunity (innate immunity) is initiated until memory of acquired immunity is completed. During this period, the compensatory change in the immune cell subgroups leads to disease recovery. During acute viral infection such as SARS and MERS, the change in the immune cell subgroups is estimated to have a significant prognosis influence. That is, the speed at which the immune cell group is compensated is estimated to be a critical factor in life-or-death situations. In FIG. 12, at the early stage of infection of an influenza infectious disease which is a clinical acute viral infectious disease, an increasing tendency of the number of NK cells and a significant decrease in the number of CD4 cells are observed, and during a recovery period, a decrease in the number of NK cells and an increase in the number of CD4 cells are observed. An amplification phenomenon (Booster role) of Th1 cell-mediated immunity due to the increase in the number of NK cells at the early stage of infection is an early initiation of natural immunity in which infected cells are removed, and during a recovery period, an increase in the number of CD4 Helper-T cells is caused by initiation of acquired immunity. Therefore, in the case of an acute viral infectious disease, an increase in the number of NK cells can be used to estimate the change in immune compensation for early recovery from infection and avoiding aggravation, in order to remove infected cells which are virus proliferation factories. That is, it is possible to accurately analyze the immune-compensation state (non-onset) and the immune-decompensation state (onset).

As described above, for a certain number of acute viral infectious disease cases, using the multi-parameter automatic blood cell counting device of the present embodiment to analyze dynamic data of the immune cell subgroups can create an appropriate treatment method (create a favorable treatment guidance) resulting in favorable prognosis without physical burden and economic burden on patients. That is, immediately ascertaining information related to three immunodynamics: (1) immune health level (normal/lowered/improved) and balance evaluation of immune autonomic nerves, (2) determination of the direction of immunity (Th1. Th2. Th17), and (3) determination in the change in immune compensation (immune-compensation state) enables to quickly and appropriately treat diseases.

While embodiments of the present disclosure have been described above in detail with reference to the drawings, the specific configuration is not limited to the embodiments and the output examples, and changes in design without departing from the scope of the present disclosure are included in the present disclosure.

**REFERENCE SIGNS LIST**

| | |
|---|---|
| 1. | Multi-parameter automatic blood cell counting device |
| 2 | Basic information generator |
| 3 | Immune cell subgroup detector |
| 4 | Immunodynamic change analyzer |
| 5 | Storage |
| 6 | Display |
| 401 | Pattern formation module |
| 402 | Immunodynamic correlator |
| 403 | Self-learning system |
| 501 | Data store |

**Claims**

1.  A multi-parameter automatic blood cell counting device (1), comprising:

    a basic information generator;
    an immune cell subgroup detector (3);
    an immunodynamic change analyzer (4);
    a storage (5); and
    a display (6),
    wherein
    the basic information generator (2) is configured to acquire examination information of a blood count of blood components and a white blood cell image, and analysis information of a CD classification of monoclonal antibodies that bind to lymphocyte surface antigens, and to store the acquired information in the storage (5),
    the immune cell subgroup detector (3) is configured to detect information of immune cell subgroups required for analyzing an immune status of a subject based on the examination information of the blood count of the blood components and the white blood cell image, and the analysis information of the CD classification of the monoclonal antibodies that bind to lymphocyte surface antigens,
    the information acquired by the basic information generator (2) and detected by the immune cell subgroup detector (3) form immune cell basic data (41) that is stored in the storage (5), and
    the immunodynamic change analyzer (4) is configured to identify at least information related to a change in immunodynamics of the subject due to administrating related cells to the subject based on the information examined in the basic information generator (2) and the immune cell subgroup detector (3), and data stored in the storage (5), and to create and display an image of the identified information on the display (6),

    wherein the immunodynamic change analyzer (4) comprises:

    a pattern former (401) generating immunodynamic patterns (42) based on the immune cell basic data (41),
    **characterized in that** the pattern former (401) immediately identifies a cell decrease pattern and a first cell compensation pattern, which are the immunodynamic pattern (42) of the subject based on the immune cell basic data (41) before administration and the immune cell basic data (41) after
    and that the immunodynamic change analyzer further comprises:
    an immunodynamic correlator (402) that provides information related to an immunodynamic correlation of the subject with respect to the immunodynamic pattern (42), wherein the immunodynamic correlator displays a graph showing the increase or decrease in the number of cells in the body of the subject based on the cell decrease pattern and the first cell compensation pattern of the subject; and
    a self-learner (403) that has a self-learning function of learning in accordance with blood count data, and that characterizes a correlation between components of the blood count.

2.  The multi-parameter automatic blood cell counting device (1) according to claim 1, wherein
    the immune cell subgroup detector (3) is configured to calculate the number of B cells, the number of NK cells, and the number of NKT cells as information required for analyzing the immune status of a subject, and to at least detect the number and the proportion of immune lymphocyte subgroups including the number of helper T cells, the number of killer T cells, the number of NK cells, the number of NKT cells, and the number of B cells.

**3.** The multi-parameter automatic blood cell counting device (1) according to claim 1 or 2, wherein

the immune cell subgroup detector (3) is configured to detect the total number of white blood cells and the number of each of immune cell subgroup as immune cells in the body of the subject based on the examination information of the blood count of the blood components and the white blood cell image, and the analysis information of the CD classification of the monoclonal antibodies that bind to the lymphocyte surface antigens, and
the immunodynamic change analyzer (4) is configured to generate an immune status pattern or an immunodynamic model based on changes in the proportion and the number of the immune cell subgroups.

**4.** The multi-parameter automatic blood cell counting device (1) according to claim 1, wherein
the self-learner (403) is configured to specify a pattern range indicating a characteristic of an infectious disease based on the quantity of total granulocytes, total lymphoid cells, CD4 antigens, CD8 antigens, NK cells, NKT cells, and B cells stored in the storage, and to store the specified range in the storage (5).

**5.** The multi-parameter automatic blood cell counting device (1) according to claim 4, wherein

the pattern former (401) is configured to create an image of the proportions of the number of CD4 antigens, the number of CD8 antigens, the number of NK cells, the number of NKT cells, and the number of B cells, stored in the storage (5), of an infected person, and to display the image on the display (6), and
information for determining whether or not the subject is infected with influenza by using comparative influenza infection pattern standard data stored in the storage (5) is displayed on the display (6).

**6.** The multi-parameter automatic blood cell counting device (1) according to any one of claims 1 to 3, wherein
the immunodynamic change analyzer (4) is configured to analyze information of the priority degree of Th1 cell-mediated immunity and Th2 humoral immunity for acquired immunity of the subject based on defined information related to the change in the immunodynamics of the subject.

**7.** The multi-parameter automatic blood cell counting device (1) according to any one of claims 1 to 3, wherein
the immunodynamic change analyzer (4) is configured to analyze an immune-compensation state and an immune-decompensation state for a clinical status of the subject based on defined information related to the change in the immunodynamics of the subject.

**8.** The multi-parameter automatic blood cell counting device (1) according to any one of claims 1 to 3, wherein
the immunodynamic change analyzer (4) is configured to analyze the priority degree of sympathetic nerves and parasympathetic nerves for autonomic nerves of the subject based on defined information related to the change in the immunodynamics of the subject.

**Patentansprüche**

**1.** Eine automatische Multiparameter-Blutzellenzählvorrichtung (1), die Folgendes beinhaltet:

einen Generator von grundlegenden Informationen;
einen Immunzellenuntergruppendetektor (3);
einen immundynamischen Änderungsanalysator (4);
einen Speicher (5); und
eine Anzeige (6),
wobei
der Generator (2) von grundlegenden Informationen konfiguriert ist, um Untersuchungsinformationen einer Blutzählung von Blutkomponenten und ein Bild von weißen Blutzellen und Analyseinformationen einer CD-Klassifizierung von monoklonalen Antikörpern, die an Lymphozytoberflächenantigene binden, zu erlangen, und die erlangten Informationen in dem Speicher (5) zu speichern,
der Immunzellenuntergruppendetektor (3) konfiguriert ist, um Informationen von Immunzellenuntergruppen zu detektieren, die erforderlich sind, um einen Immunstatus einer Testperson basierend auf den Untersuchungsinformationen der Blutzählung der Blutkomponenten und des Bilds von weißen Blutzellen und den Analyseinformationen der CD-Klassifizierung der monoklonalen Antikörper, die an Lymphozytoberflächenantigene binden, zu analysieren,
die Informationen, die durch den Generator (2) von grundlegenden Informationen erlangt und durch den Immunzellenuntergruppendetektor (3) detektiert werden,

grundlegende Daten (41) von Immunzellen bilden, die in dem Speicher (5) gespeichert werden, und

der immundynamische Änderungsanalysator (4) konfiguriert ist, um mindestens Informationen, die sich auf eine Änderung in Immundynamik der Testperson aufgrund des Verabreichens zugehöriger Zellen an die Testperson beziehen, basierend auf den Informationen, die in dem Generator (2) von grundlegenden Informationen und dem Immunzellenuntergruppendetektor (3) untersucht werden und auf Daten, die in dem Speicher (5) gespeichert sind, zu identifizieren und ein Bild der identifizierten Informationen zu kreieren und auf der Anzeige (6) anzuzeigen,

wobei der immundynamische Änderungsanalysator (4) Folgendes beinhaltet:

einen Musterbildner (401), der immundynamische Muster (42) basierend auf den grundlegenden Daten (41) von Immunzellen erzeugt,

**dadurch gekennzeichnet, dass** der Musterbildner (401) ein Zellverminderungsmuster und ein erstes Zellkompensationsmuster unmittelbar identifiziert, die das immundynamische Muster (42) der Testperson basierend auf den grundlegenden Daten (41) von Immunzellen vor der Verabreichung und den grundlegenden Daten (41) von Immunzellen danach sind,

und dass der immundynamische Änderungsanalysator ferner Folgendes beinhaltet:

einen immundynamischen Korrelator (402), der Informationen bereitstellt, die sich auf eine immundynamische Korrelation der Testperson mit Bezug auf das immundynamische Muster (42) beziehen, wobei der immundynamische Korrelator ein Diagramm anzeigt, das die Erhöhung oder Verminderung in der Zahl von Zellen in dem Körper der Testperson basierend auf dem Zellenverminderungsmuster und dem ersten Zellkompensationsmuster der Testperson darstellt; und

einen Selbst-Lerner (403), der gemäß Blutzählungsdaten eine Selbstlernfunktion aufweist und der eine Korrelation zwischen Komponenten der Blutzählung charakterisiert.

2. Automatische Multiparameter-Blutzellenzählvorrichung (1) gemäß Anspruch 1, wobei der Immunzellenuntergruppendetektor (3) konfiguriert ist, um die Zahl von B-Zellen, die Zahl von NK-Zellen und die Zahl von NKT-Zellen als Information zu berechnen, die erforderlich ist, um den Immunstatus einer Testperson zu analysieren, und mindestens die Zahl und den Anteil von Immunlymphozytuntergruppen, die die Zahl von Helfer-T-Zellen, die Zahl von Killer-T-Zellen, die Zahl von NK-Zellen, die Zahl von NKT-Zellen und die Zahl von B-Zellen umfassen, zu detektieren.

3. Automatische Multiparameter-Blutzellenzählvorrichung (1) gemäß Anspruch 1 oder 2, wobei

der Immunzellenuntergruppendetektor (3) konfiguriert ist, um die Gesamtzahl von weißen Blutzellen und die Zahl von jeder Immunzellenuntergruppe als Immunzellen in dem Körper der Testperson basierend auf den Untersuchungsinformationen der Blutzählung der Blutkomponenten und des Bilds von weißen Blutzellen und den Analyseinformationen der CD-Klassifizierung der monoklonalen Antikörper, die an die an Lymphozytoberflächenantigene binden, zu detektieren, und

der immundynamische Änderungsanalysator (4) konfiguriert ist, um ein Immunstatusmuster oder ein immundynamisches Modell basierend auf Änderungen in dem Anteil und der Zahl der Immunzellenuntergruppen zu erzeugen.

4. Automatische Multiparameter-Blutzellenzählvorrichung (1) gemäß Anspruch 1, wobei der Selbst-Lerner (403) konfiguriert ist, um einen Musterbereich zu spezifizieren, der eine Charakteristik einer Infektionskrankheit basierend auf der Menge von Gesamtgranulocyten, Gesamtlymphoidzellen, CD4-Antigenen, CD8-Antigenen, NK-Zellen, NKT-Zellen und B-Zellen, die in dem Speicher gespeichert sind, angibt und den spezifizierten Bereich in dem Speicher (5) zu speichern.

5. Automatische Multiparameter-Blutzellenzählvorrichung (1) gemäß Anspruch 4, wobei der Musterbildner (401) konfiguriert ist, um ein Bild der Anteile der Zahl von CD4-Atigenen, der Zahl von CD8-Antigenen, der Zahl von NK-Zellen, der Zahl von NKT-Zellen und der Zahl von B-Zellen einer infizierten Person, gespeichert in dem Speicher (5), zu kreieren und das Bild auf der Anzeige (6) anzuzeigen, und

Informationen zum Bestimmen, ob die Testperson mit Influenza infiziert ist oder nicht, durch das Verwenden von Vergleichsmusterstandarddaten von Influenzainfektion, die in dem Speicher (5) gespeichert ist, auf der Anzeige (6) angezeigt werden.

6. Automatische Multiparameter-Blutzellenzählvorrichung (1) gemäß einem der Ansprüche 1 bis 3, wobei

der immundynamische Änderungsanalysator (4) konfiguriert ist, um Informationen des Prioritätsgrads von zellulärer Th1-Immunität und humoraler Th2-Immunität für erlangte Immunität der Testperson basierend auf den definierten Informationen, die sich auf die Änderung in der Immundynamik der Testperson beziehen, zu analysieren.

**7.** Automatische Multiparameter-Blutzellenzählvorrichtung (1) gemäß einem der Ansprüche 1 bis 3, wobei der immundynamische Änderungsanalysator (4) konfiguriert ist, um einen Immunkompensationszustand und einen Immundekompensationszustand für einen klinischen Zustand der Testperson basierend auf den definierten Informationen, die sich auf die Änderung in der Immundynamik der Testperson beziehen, zu analysieren.

**8.** Automatische Multiparameter-Blutzellenzählvorrichtung (1) gemäß einem der Ansprüche 1 bis 3, wobei der immundynamische Änderungsanalysator (4) konfiguriert ist, um den Prioritätsgrad von sympathischen Nerven und parasympathischen Nerven für autonome Nerven der Testperson basierend auf den definierten Informationen, die sich auf die Änderung in der Immundynamik der Testperson beziehen, zu analysieren.

**Revendications**

**1.** Un dispositif de numération de cellules sanguines automatique multi-paramètre (1), comprenant :

un générateur d'informations de base ;
un détecteur de sous-groupes de cellules immunitaires (3) ;
un analyseur de changement immunodynamique (4) ;
une unité de stockage (5) ; et
une unité d'affichage (6),
dans lequel
le générateur d'informations de base (2) est configuré pour acquérir des informations d'examen d'une numération sanguine de composants sanguins et une image de globules blancs, et des informations d'analyse d'une classification CD d'anticorps monoclonaux qui se lient à des antigènes de surface lymphocytaires, et pour stocker les informations acquises dans l'unité de stockage (5),
le détecteur de sous-groupes de cellules immunitaires (3) est configuré pour détecter des informations de sous-groupes de cellules immunitaires requises pour l'analyse d'un statut immunitaire d'un sujet sur la base des informations d'examen de la numération sanguine des composants sanguins et de l'image de globules blancs, et des informations d'analyse de la classification CD des anticorps monoclonaux qui se lient à des antigènes de surface lymphocytaires,
les informations acquises par le générateur d'informations de base (2) et détectées par le détecteur de sous-groupes de cellules immunitaires (3) forment des données de base de cellules immunitaires (41) qui sont stockées dans l'unité de stockage (5), et
l'analyseur de changement immunodynamique (4) est configuré pour identifier au moins des informations relatives à un changement de l'immunodynamique du sujet en raison de l'administration de cellules connexes au sujet sur la base des informations examinées dans le générateur d'informations de base (2) et le détecteur de sous-groupes de cellules immunitaires (3), et de données stockées dans l'unité de stockage (5), et pour créer et afficher une image des informations identifiées sur l'unité d'affichage (6),
dans lequel l'analyseur de changement immunodynamique (4) comprend :

une unité de formation de motifs (401) générant des motifs immunodynamiques (42) sur la base des données de base de cellules immunitaires (41),
**caractérisé en ce que** l'unité de formation de motifs (401) identifie immédiatement un motif de diminution de cellules et un premier motif de compensation de cellules, qui sont le motif immunodynamique (42) du sujet sur la base des données de base de cellules immunitaires (41) avant administration et des données de base de cellules immunitaires (41) après
et **en ce que** l'analyseur de changement immunodynamique comprend en outre :

un corrélateur immunodynamique (402) qui fournit des informations relatives à une corrélation immunodynamique du sujet par rapport au motif immunodynamique (42), le corrélateur immunodynamique affichant un graphique montrant l'augmentation ou la diminution du nombre de cellules dans le corps du sujet sur la base du motif de diminution de cellules et du premier motif de compensation de cellules du sujet ; et
une unité d'auto-apprentissage (403) qui a une fonction d'auto-apprentissage d'apprentissage conformément à des données de numération sanguine, et qui caractérise une corrélation entre des composants de la numération sanguine.

2. Le dispositif de numération de cellules sanguines automatique multi-paramètre (1) selon la revendication 1, dans lequel

le détecteur de sous-groupes de cellules immunitaires (3) est configuré pour calculer le nombre de cellules B, le nombre de cellules tueuses naturelles, et le nombre de cellules T tueuses naturelles comme informations requises pour l'analyse du statut immunitaire d'un sujet, et pour au moins détecter le nombre et la proportion de sous-groupes de lymphocytes immunitaires incluant le nombre de cellules T auxiliaires, le nombre de cellules T tueuses, le nombre de cellules tueuses naturelles, le nombre de cellules T tueuses naturelles, et le nombre de cellules B.

3. Le dispositif de numération de cellules sanguines automatique multi-paramètre (1) selon la revendication 1 ou la revendication 2, dans lequel

le détecteur de sous-groupes de cellules immunitaires (3) est configuré pour détecter le nombre total de globules blancs et le nombre de chacun des sous-groupes de cellules immunitaires comme cellules immunitaires dans le corps du sujet sur la base des informations d'examen de la numération sanguine des composants sanguins et de l'image de globules blancs, et des informations d'analyse de la classification CD des anticorps monoclonaux qui se lient aux antigènes de surface lymphocytaires, et l'analyseur de changement immunodynamique (4) est configuré pour générer un motif de statut immunitaire ou un modèle immunodynamique sur la base de changements de la proportion et du nombre des sous-groupes de cellules immunitaires.

4. Le dispositif de numération de cellules sanguines automatique multi-paramètre (1) selon la revendication 1, dans lequel

l'unité d'auto-apprentissage (403) est configurée pour spécifier une gamme de motifs indiquant une caractéristique d'une maladie infectieuse sur la base de la quantité de granulocytes totaux, de cellules lymphoïdes totales, d'antigènes CD4, d'antigènes CD8, de cellules tueuses naturelles, de cellules T tueuses naturelles, et de cellules B stockés dans l'unité de stockage, et pour stocker la gamme spécifiée dans l'unité de stockage (5).

5. Le dispositif de numération de cellules sanguines automatique multi-paramètre (1) selon la revendication 4, dans lequel

l'unité de formation de motifs (401) est configurée pour créer une image des proportions du nombre d'antigènes CD4, du nombre d'antigènes CD8, du nombre de cellules tueuses naturelles, du nombre de cellules T tueuses naturelles, et du nombre de cellules B, stockés dans l'unité de stockage (5), d'une personne infectée, et pour afficher l'image sur l'unité d'affichage (6), et

des informations pour la détermination de si oui ou non le sujet est infecté par la grippe en utilisant des données standard de motif d'infection par la grippe comparatives stockées dans l'unité de stockage (5) sont affichées sur l'unité d'affichage (6).

6. Le dispositif de numération de cellules sanguines automatique multi-paramètre (1) selon l'une quelconque des revendications 1 à 3, dans lequel

l'analyseur de changement immunodynamique (4) est configuré pour analyser des informations du degré de priorité d'immunité à médiation cellulaire de type Th1 et d'immunité humorale de type Th2 pour l'immunité acquise du sujet sur la base d'informations définies relatives au changement de l'immunodynamique du sujet.

7. Le dispositif de numération de cellules sanguines automatique multi-paramètre (1) selon l'une quelconque des revendications 1 à 3, dans lequel

l'analyseur de changement immunodynamique (4) est configuré pour analyser un état de compensation immunitaire et un état de décompensation immunitaire pour un statut clinique du sujet sur la base d'informations définies relatives au changement de l'immunodynamique du sujet.

8. Le dispositif de numération de cellules sanguines automatique multi-paramètre (1) selon l'une quelconque des revendications 1 à 3, dans lequel

l'analyseur de changement immunodynamique (4) est configuré pour analyser le degré de priorité de nerfs sympathiques et de nerfs parasympathiques pour les nerfs du système autonome du sujet sur la base d'informations définies relatives au changement de l'immunodynamique du sujet.

FIG. 1

Multi-parameter automatic blood cell counting device 1

Display 6 ⟷ Immune cell subgroup detector 3

Storage 5

Immunodynamic change analyzer 4
Pattern formation module 401
Immunodynamic correlator 402
Self-learning system 403

Basic information generator 2
Blood cell count detector 21
Surface antigen CD number detector 22

FIG. 2

EP 4 160 183 B1

| Item name | | Result | Standard Value |
|---|---|---|---|
| WBC | | | 3500~9700 μL |
| RBC | | | M 438~577 (×104/μL)<br>F 376~516 (×104/μL) |
| Hemoglobin (Hb) | | | M 13.6~18.3 (g/dL)<br>F 11.2~15.2 (g/dL) |
| Hematocrit (Ht) | | | M 40.4~50.9 (%)<br>F 34.3~45.2 (%) |
| MVC | | | M 83~101<br>F 80~101 |
| MCH | | | M 28.2~34.7<br>F 26.4~34.3 |
| MCHC | | | M 31.8~36.4<br>F 31.3~36.1 |
| Platelet | | | M14.0 ~37.9(x104/μL) |
| Reticulocyte | | | 0.1~2.6 |
| WBC image | Baso | | 0.0~2.0% |
| | Eosino | | 0.0~7.0 |
| | Neutro | | 42~74% |
| | Stab | | 0.0~19.0% |
| | Seg | | 27.0~72.0% |
| | Mono | | 1.0~8.0% |
| | Lympho | | 18.0~50.0% |
| | Other 1 | | |
| | Other 2 | | |
| | EBL (erythroblast) | | |
| RBC morphology | anisocytosis | | (−) |
| | deformity | | (−) |
| | pleochromia | | (−) |
| | nucleated | | (−) |
| Blood gravity | | | M 10.55~1.063<br>F 1.052~1.060 |

(Essential labo. Data)

・Blood calculation result
・WBC demarcation
・Lymphocyte differentiation
 (CD3, CD4, CD8, CD16, CD56)

| Item name | | Result | Standard Value |
|---|---|---|---|
| WBC | | | 3500~9700 μL |
| RBC | | | M 438~577 (×104/μL)<br>F 376~516 (×104/μL) |
| Hemoglobin (Hb) | | | M 13.6~18.3 (g/dL)<br>F 11.2~15.2 (g/dL) |
| Hematocrit (Ht) | | | M 40.4~50.9 (%)<br>F 34.3~45.2 (%) |
| MVC | | | M 83~101<br>F 80~101 |
| MCH | | | M 28.2~34.7<br>F 26.4~34.3 |
| MCHC | | | M 31.8~36.4<br>F 31.3~36.1 |
| Platelet | | | M14.0 ~37.9(x104/μL) |
| Reticulocyte | | | 0.1~2.6 |
| WBC image | Baso | | 0.0~2.0% |
| | Eosino | | 0.0~7.0 |
| | Neutro | | 42~74% |
| | Stab | | 0.0~19.0% |
| | Seg | | 27.0~72.0% |
| | Mono | | 1.0~8.0% |
| | Lympho | | 18.0~50.0% |
| Lymphocyte subgroups | Helper T cell | | CD3+　CD4+ |
| | Killer T cell | | CD3+　CD8+ |
| | NK cell | | CD3−　CD56+ or CD16+ |
| | NKT cell | | CD3+　CD56+ |
| | B cell | | CD3−　CD56− or CD16− |
| | (Total T cell) | | CD3+ |
| | (Total non-T cell) | | CD3− |
| | Other 1 | | |
| | Other 2 | | |
| | EBL(erythroblast) | | |
| RBC morphology | anisocytosis | | (−) |
| | deformity | | (−) |
| | pleochromia | | (−) |
| | nucleated | | (−) |
| Blood gravity | | | M 10.55~1.063<br>F 1.052~1.060 |

FIG. 3A

IMMUNODYNAMIC PATTERN 42

LYMPHOCYTE FRACTION

FIG. 3B

IMMUNODYNAMIC MODEL 43

FIG. 4

Immunodynamic change analyzer 4

Pattern formation module 401

Immunodynamic correlator 402

Self-learning system 403

Data store 501

FIG. 5

FIG. 6A

| INFLUENZA INFECTION PATTERNS | AVE. VALUE | MAX. VALUE | MIN. VALUE |
|---|---|---|---|
| CD4 | 21% | 14% | 29% |
| CD8 | 22% | 13% | 37% |
| NK | 16% | 27% | 9% |
| NKT | 4% | 2% | 7% |
| B | 37% | 20% | 51% |
| Body temperature (°C) | 38°C or more | | |
| CD4/B | 0.59 | | |
| Total lymphoid cells/ total granules | 0.13 | | |

AVERAGE VALUES OF INFLUENZA INFECTION PATTERNS

21%

37%

22%

4%

16%

■1
■2
■3
■4
■5

FIG. 6B

| IMMUNE CELL BASIC DATA OF SUBJECT | NUMBERS | PROPORTIONS |
|---|---|---|
| CD4 | 40 | 17% |
| CD8 | 84 | 37% |
| NK | 43 | 19% |
| NKT | 15 | 7% |
| B | 46 | 20% |
| Body temperature (°C) | 40 | |
| CD4/B | 0. 87 | |
| Total lymphoid cells/ total granules | 0. 11 | |

IMMUNE CELL BASIC DATA OF SUBJECT

FIG. 7

FIG. 8

FIG. 9

| | PATIENT WITH ESOPHAGEAL CANDIDA | PATIENT WITH PSORIASIS |
|---|---|---|
| NK ( #$10^7$) | 129 | 25.3 |
| CD$_8$( #$10^7$) | 173 | 76 |
| CD$_4$ ( #$10^7$) | 132 | 416 |
| NKT ( #$10^7$) | 16.5 | 3 |
| B cell ( #$10^7$) | 271 | 155 |
| Mono ( #$10^7$) | 148 | 206 |
| Granular ( #$10^7$) | 1786 | 1833 |
| T cell ( #$10^7$) | 500 | 600 |
| Th1/Lym (%) | 42 | 15 |
| Th2/Lym (%) | 58 | 85 |
| B cell/Lym (%) | 38 | 23 |

FIG. 10

FIG. 11

| | BEFORE NK CELL INJECTION | AFTER NK CELL INJECTION |
|---|---|---|
| NK ( #10$^7$) | 66 | 46 |
| CD$_8$ ( #10$^7$) | 264 | 240 |
| CD$_4$ ( #10$^7$) | 274 | 329 |
| NKT ( #10$^7$) | 17 | 46 |
| B cell ( # 10$^7$) | 202 | 183 |
| Mono ( #10$^7$) | 141 | 149 |
| Granular ( #10$^7$) | 2038 | 1828 |
| T cell ( #10$^7$) | 682 | 703 |
| Th1/Lym (%) | 40 | 34 |
| Th2/Lym (%) | 60 | 66 |
| B cell/Lym (%) | 23 | 22 |

FIG. 12

EP 4 160 183 B1

CHANGES IN IMMUNE CELL GROUP
DURING INFMECTION PERDIOD AND
REVOVERY PERIOD IN INFLUENZA CASE

**INFLUENZA A INFECTION PERIOD**

| | (10 millions) |
|---|---|
| Total white blood cells | 2990 |
| Total granulocytes | 2545 |
| Total Monocytes | 176 |
| Total lymphocytes | 269 |
| Total T cells | 172 |
| Total non-T cells | 97 |
| Total CD4 | 40 |
| Total CD8 | 84 |
| Total NK cells | 43 |
| Total NKT cells | 15 |
| Total B cells | 46 |

**1/21/2013**

DURING INFLUENZA INFECTION

Body temp: 40.0. C
Pulse rate:118/min
Influenza A positive
WBC  5980
CRP  1.05
CPK 240

CD8 3% B cell 2% NK cell 1% NKT cell 1%
CD4 1%
Mono 6%
Granular 86%

NKT cell 4% B cell 11%
NK cell 11%
Mono 43%
CD8 21%
CD4 10%

•GRANULOCYTES: MONONUCLEAR CELLS: LYMPHOCYTES = **86 : 6 : 8**%
•CD4 IMMUNITY CENTER = **80** / μl
•NK CELL NUMBERS = 430 MILLIONS, NK CELL PROPORTION = 19%

B cell 20% CD4 17%
NKT cell 7%
NK cell 19% CD8 37%

**RECOVERY FROM INFLUENZA A**

| | (10 millions) |
|---|---|
| Total white blood cells | 2205 |
| Total granulocytes | 1475 |
| Total Monocytes | 265 |
| Total lymphocytes | 465 |
| Total T cells | 352 |
| Total non-T cells | 113 |
| Total CD4 | 117 |
| Total CD8 | 147 |
| Total NK cells | 27 |
| Total NKT cells | 30 |
| Total B cells | 76 |

**1/24/2013**

WHEN RECOVERED FROM INFLUENZA

Body temp: 37.4. C
Pulse rate: 78/min
Influenza A positive
WBC  4410

CD8 NK cell 1% NKT cell 1% B cell 4%
CD4 7%
6%
Mono 12%
Granular 69%

NKT cell 5% B cell 11%
NK cell 4%
Mono 40%
CD8 22%
CD4 18%

•GRANULOCYTES: MONONUCLEAR CELLS: LYMPHOCYTES = **69 : 12 : 19**%
•CD4 IMMUNITY CENTER = **234** / μl
•NK CELL NUMBERS = 270 MILLIONS, NK CELL PROPORTION = 7%

B cell 19% CD4 29%
NKT cell 8%
NK cell 7% CD8 37%

36

FIG. 13

* GRANULOCYTE GROUP
(NEUTROPHILS, BASOPHILS, EOSINOPHILS)
→ NATURAL IMMUNE CELLS
* MONONUCLEAR CELL GROUP
→ NATURAL IMMUNE CELLS
* LYMPHOCYTE GROUP
· KILLER CELL SUBGROUP → ADAPTIVE IMMUNE CELLS
· HELPER T CELL SUBGROUP → ADAPTIVE IMMUNE CELLS
· B CELL SUBGROUP → ADAPTIVE IMMUNE CELLS
· NK CELL SUBGROUP → EARLY INDUCTION REACTION CELLS
· NKT CELL SUBGROUP → EARLY INDUCTION REACTION CELLS

**ASCERTAIN NUMBERS**
**AND PROPORTIONS OF**
**IMMUNE CELL SUBGROUPS**

IMMUNITY RESTRAIN

Treg

Th17 cell
(Intestinal immunity) ⬅ CD4 — Th2 cell — B cell ➡ HUMORAL IMMUNITY ↑

NKT cell

NK cell → Th1 cell

CELLULAR IMMUNITY
(killer-T=CD8)

·Th1 Cellular Immunity = NK+CD8 (killer-T)
·Th2 Humoral Immunity = NKT+B cell

**CLINICAL**
**APPLICATION**
* DIAGNOSIS OF INTERNAL IMMUNITY HEALTH LEVEL
* DIAGNOSIS OF INTERNAL IMMUNITY FLOW
* AUXILIARY DIAGNOSIS OF EARLY IMMUNE ABNORMANITY
(EARLY DETECTION OF IMMUNE IMPAIRED)
* DIAGNOSIS MEDICATION AND TREATMENT EFFECT OF EACH DISEASE
(DIAGNOSIS OF TREATMENT EFFECT AND SIDE EFFECT)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018259504 A1
- JP 2008089382 A
- WO 2007145333 A1